(19) Europäisches Patentamt / European Patent Office / Office européen des brevets

(11) **EP 4 353 244 A1**

(12) **EUROPEAN PATENT APPLICATION**
published in accordance with Art. 153(4) EPC

(43) Date of publication:
**17.04.2024 Bulletin 2024/16**

(51) International Patent Classification (IPC):
**A61K 35/28** (2015.01)        **A61K 35/30** (2015.01)
**A61P 25/28** (2006.01)        **C12N 5/079** (2010.01)

(21) Application number: **23833292.8**

(22) Date of filing: **03.05.2023**

(86) International application number:
**PCT/KR2023/006022**

(87) International publication number:
**WO 2024/048900 (07.03.2024 Gazette 2024/10)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: 30.08.2022  KR 20220109087
              23.02.2023  KR 20230024598

(71) Applicants:
• **Seoul National University R & DB Foundation**
  **Seoul 08826 (KR)**
• **Industry - University Cooperation Foundation**
  **Hanyang University**
  **04763 Seoul (KR)**

(72) Inventors:
• **CHANG, Mi Sook**
  **Seoul 03080 (KR)**
• **KOH, Seong Ho**
  **Seoul 04763 (KR)**
• **LEE, Eun Ji**
  **Seoul 04763 (KR)**

(74) Representative: **Stolmár & Partner**
  **Patentanwälte PartG mbB**
  **Blumenstraße 17**
  **80331 München (DE)**

Remarks:
The complete document including Reference
Table(s) and the Sequence Listing(s) can be
downloaded from the EPO website

(54) **PHARMACEUTICAL COMPOSITION FOR PREVENTING OR TREATING VASCULAR DEMENTIA**

(57)    The present invention provides a pharmaceutical composition for prevention or treatment of vascular dementia, which includes glia-like cells differentiated from human mesenchymal stem cells (hMSCs). The pharmaceutical composition of the present invention preferably induces the regeneration of cerebral cells or glia cells or inhibits further damage, such that the composition can be used as a therapeutic agent for preventing or treating vascular dementia.

EP 4 353 244 A1

[FIG. 6b]

*p<0.01 by ANOVA and Tukey's post hoc vs Control
**p<0.0001 by ANOVA and Tukey's post hoc vs Control

**Description**

[Technical Field]

The present invention relates to a pharmaceutical composition for prevention or treatment of vascular dementia, which includes glia-like cells differentiated from human mesenchymal stem cells (hMSCs).

[Background Art]

In 2017, 65 aged population in Korea accounts for more than 14% and enters an aging society. Korea is a country that enters the most rapidly the aging society (2017) and will enter a super high aged society (2026). Thus, expenses of social and economic costs due to this reason are now very quickly increasing. Along with population aging, the number of patients suffering from different geriatric diseases including dementia and stroke is also rapidly increasing. Henceforth, it is expected that the number of patients would be further rapidly increased. Vascular dementia is the second most common cause for dementia after Alzheimer's disease, however, if even the vascular dementia mixed with Alzheimer's disease is included, it is presumed that the vascular dementia may be very common as a cause of the senile dementia. In relation to the vascular dementia, antithrombotics, ergot alkaloids, nootropics, thyrotropin-releasing pseudo-hormones, ginkgo bioba extract, medicines related to plasma viscosity, high pressure oxygen therapy, antioxidants, serotonin and histamine receptor agonists, vasoactive drugs, xanthine derivatives, calcium agonists, diabetes therapeutics, statin, deep brain stimulation, etc. have been tested. However, there is still no particular vascular dementia therapeutic agent approved around the world.

In regard to the reason of failures in numerous clinical experiments for development of vascular dementia therapeutics executed till the present, a number of experts presume that the above reason is why some therapeutic agents purposed of a single target have been developed in relation to the variety of types of vascular dementia and complicated and diverse pathogenic mechanisms, and some clinical tests were executed using the above therapeutic agents. Accordingly, a stem cell therapeutic agent involving different mechanisms concurrently emerges as a preferable alternative. In addition to dementia area, the stem cell therapeutic agents are now being developed as therapeutics for different intractable diseases, and a market scale thereof is being increased geometrically. Although intrinsic neural stem cells exist in subventricular portion of the brain and may participate in neural regeneration after brain disease, such cases are absolutely few and entail limitation in regeneration ability. Therefore, a number of studies intended to induce neural regeneration *in vivo* through cell treatment using peripheral neural stem cells, neural precursor cells, mesenchymal stem cells (MSCs), etc., is being carried out.

The stem cell can be classified in various ways, however, adult stem cells having the highest safety are mostly used for clinical studies. First generation adult stem cell therapeutics used now cannot be used in clinical phase due to high-cost and low-efficacy and, when it is administered to patients with intractable neural diseases, effects thereof is insignificant. Due to these disadvantages, it is difficult to satisfy NIH, FDA guideline STEPS3 international standards, and it is expected that it will be difficult to enter the global market. Among the various stem cells, specifically, mesenchymal stem cells are used to enhance therapeutic efficacy, and then, transplanted in the body to increase the survival rate of stem cells. Further, some studies for inducing differentiation of the stem cells into nerve cells or glia cells, and thus inducing neuron regeneration and functional recovery thereof are being carried out. Researches oversea are now preparing to use the next generation adult stem cells with therapeutic efficacy reinforced by introducing a functional gene into the adult stem cells in clinical trials for treatment of intractable neural diseases. In this case, it may cause a problem of safety in the field of gene operation. Therefore, it is currently necessary to research a stem cell therapeutic agent, which not only ensures medical efficacy on vascular dementia and but also is free from safety and moral problems.

[Summary of Invention]

[Problems to be Solved by Invention]

An object of the present invention is to provide a pharmaceutical composition for prevention or treatment of vascular dementia, which includes glia-like cells.

[Means for Solving Problems]

    1. A pharmaceutical composition for prevention or treatment of vascular dementia, including glia-like cells differentiated from human mesenchymal stem cells (hMSCs).

2. The pharmaceutical composition according to the above 1, wherein the glia-like cell expresses one or more marker gene selected from the group consisting of GFAP, GLAST, HGF, VEGF, IGFBP-4 and PAPP-A.

3. The pharmaceutical composition according to the above 1, wherein the human mesenchymal stem cell is derived from tissues selected from the group including bone marrow, fat, peripheral blood, cord blood, muscle and skin.

4. The pharmaceutical composition according to the above 1, wherein the human mesenchymal stem cell was passage-cultured twice to 15 times.

[Advantageous Effects]

The pharmaceutical composition for prevention or treatment of vascular dementia, which includes glia-like cells differentiated from human mesenchymal stem cells, may induce regeneration of cerebral cells or glia cells in the damaged brain portion or inhibit damage thereto. Therefore, this composition may be used for inhibiting the occurrence of vascular dementia or for alleviating or treating the progress of dementia.

[Brief Description of Drawings]

FIG. 1 illustrates confirmation of morphological variation of glia-like cells during induction thereof, and an expression level of a marker gene of the glia cell.
FIG. 2 is a schematic view illustrating an experimental plan in order to identify whether vascular dementia is treated or not by the glia-like cells.
FIG. 3 is a schematic view indicating a blocked site in the common carotid arterial stenosis operation in order to induce vascular dementia.
FIG. 4 illustrates tissue analysis data in a lesion site of corpus callosum or internal capsule.
FIG. 5 illustrates pyknotic neuron staining data in CA1 and CA2 of the hippocampus, and the number of pyknotic neurons based on the total neurons in CA1 and CA2 of the hippocampus.
FIG. 6 illustrates a process of water maze experiment depending on whether to treat with glia-like cells or not after induction of vascular dementia, and analysis data thereof.
FIG. 7 illustrates a process of Y maze experiment depending on whether to treat with glia-like cells or not after induction of vascular dementia, and analysis data thereof.
FIG. 8 illustrates analysis data of water maze experiment depending on whether to treat with glia-like cells or not after induction of vascular dementia, and analysis data thereof.
FIG. 9 illustrates analysis data of water maze experiment depending on whether to treat with glia-like cells or adult stem cells after induction of vascular dementia.
FIG. 10 illustrates ELISA results of hMSC and ghMSC.
FIG. 11 illustrates results of flow cytometry of ghMSC and astrocytes.
FIG. 12 illustrates results of cytokine array of hMSC and ghMSC.
FIG. 13 is an experimental schematic view for confirming ghMSC-CM protection effects of mouse brain endothelial cells (bEnd.3 cells), and analysis data thereof.
FIG. 14 is an experimental schematic view for confirming organotypic brain (Hippocampus) slice culture and analysis data thereof, and illustrates results of evaluating cell protection ability in an oxygen-glucose deprivation (OGD)-induced rat brain (Hippocampus) injury model.
FIG. 15 illustrates results of confirming neuron marker in an oxygen-glucose deprivation (OGD)-induced rat brain (Hippocampus) injury model.
FIG. 16 illustrates results of qRT-PCR of hMSC and ghMSC.

[Mode for Carrying out Invention]

Hereinafter, the present invention will be described in detail. Unless otherwise specifically defined, all terms in the present specification would have the same meanings as general meanings of the corresponding terms understood by persons having common knowledge to which the present invention pertains ("those skilled in the art"), and if the general meanings conflict with the meanings of the terms used herein, the meanings used in the present specification take precedence.

The present invention relates to a pharmaceutical composition for prevention or treatment of vascular dementia, which includes glia-like cells differentiated from human mesenchymal stem cell (hMSC).

In the present invention, the glia-like cell corresponds to a cell expressing a marker gene of glia cell, in particular, astrocyte, or a cell secreting growth factor or cytokine secreted from the astrocyte. The marker gene may correspond to GFAP, GLAST, HGF, VEGF, IGFBP-4 or PAPP-A, while the glia-like cell may refer to cells relatively highly expressing at least one or more among the marker genes, as compared to undifferentiated stem cells. The lower limit of mRNA expression level of the marker gene in the glia-like cells may be 1.1, 1.2, 1.3, 1.5 or 2 times as compared to the undifferentiated stem cells, while the upper limit of mRNA expression level of the marker gene in the glia-like cells may be 20, 15, 10, 8, 6 or 3 times as compared to the undifferentiated stem cells, but they are not limited thereto.

The glia-like cells according to the present invention are differentiated from hMSCs, and any glia-like cell is included in the scope of the present invention without limitation thereof in terms of the preparation method, so far as it can express the marker gene. Examples of the preparation method may include a process consisting of: primary culture of hMSCs in a medium containing β-mercaptoethanol; secondary culture of the primary culture product in a medium containing all-trans-retinoic acid; and tertiary culture of the secondary culture product in a medium containing human basic fibroblast growth factor (hbFGF), human platelet derived growth factor AA (hPDGF-AA), forskolin and heregulin-β1 (HRG-β1) to produce the final product, but they are not limited thereto.

In the primary culture, the mercaptoethanol may be included in an amount of 0.05 to 20 mM, 0.1 to 10 mM, 0.2 to 5 mM or 0.5 to 2 mM based on the total primary culture solution. Further, in the secondary culture, the all-trans-retinoic acid may be included in an amount of 0.01 to 2 μg/mL, 0.05 to 1 ug/mL or 0.1 to 0.5 μg/mL based on the total secondary culture solution. Further, in the tertiary culture, the human basic fibroblast growth factor (hbFGF) may be included in an amount of 0.01 to 1 ng/mL, 0.1 to 100 ng/mL, 1 to 50 ng/mL or 5 to 20 ng/mL based on the total tertiary culture solution. Likewise, the human platelet derived growth factor AA (hPDGF-AA) may be included in an amount of 0.1 to 100 ng/mL, 0.5 to 20 ng/mL or 1 to 10 ng/mL, the forskolin may be included in an amount of 0.5 to 100 μM, 1 to 50 μM or 5 to 20 μM, and the heregulin-β1 (HRG-β1) may be included in an amount of 1 ng/mL to 1 μg/mL, 10 to 500 ng/mL or 50 to 300 ng/mL, but they are not limited thereto. In addition, the culture solution may further include a composition such as DMEM or FBS to provide nutrients commonly used in cell culture, antibacterial/antiviral substances such as a mixture of penicillin/streptomycin, or the like, but it is not limited thereto.

In the present invention, the vascular dementia refers to a chronic disease occurring such that brain blood vessels are slowly blocked over a long time, and cerebral blood flow is chronically reduced, which in turn causes cerebral neural cells to slowly dead and do not function. The vascular dementia is dementia that can gradually occur due to some causes such as hypertension, diabetes, hyperlipidemia, heart disease, smoking, obesity, lack of exercise, or the like, and corresponds to a disease distinguishable from Alzheimer's dementia occurring due to an accumulation of amyloid-β.

The pharmaceutical composition of the present invention may promote the regeneration of neural stem cells that may be differentiated into corresponding cerebral cells at a site in which the cerebral cells including nerve cells, astrocytes or oligodendrocytes are damaged and/or dead, or induce improvement of brain functions, whereby the vascular dementia can be prevented or treated.

As the human mesenchymal stem cell (hMSC) in the present invention, any hMSC may be included in the scope of the present invention regardless of tissues from which the hMSC is derived so far as it can be induced into glia-like cell by the culture process as described above. For example, it may be derived from tissues selected from the group including bone marrow, fat, peripheral blood, cord blood, muscle and skin, but it is not limited thereto.

The hMSC may be differentiated into glia-like cells and used in the present invention regardless of the number of passage cultures. For example, primary culture line may be used, or hMSCs passage-cultured once or more may be used. Further, even the late-passage stem cells, which were passage-cultured 9 times or more, may be induced into glia-like cells showing effects of alleviating the cerebral infarction sequela described above. Specifically, the lower limit of the number of passage cultures is not limited but may be 1, 2, 3 or 4 times. On the other hand, the upper limit of the number of passage cultures may be 20, 15, 12, 11, 10, 9 or 8 times, but it is not limited thereto.

The pharmaceutical composition of the present invention may include active ingredients alone, or further include at least one pharmaceutically acceptable carrier, excipient or diluent to be provided as a pharmaceutical composition. Further, a substance having similar efficacy known in the art may also be administered in combination with the cells, but it is not limited thereto.

An administration route of the pharmaceutical composition according to the present invention may include oral, intravenous, intramuscular, intra-arterial, intra-medullary, intradural, intracardiac, transdermal, subcutaneous, intraperitoneal, intranasal, intestinal, local, sublingual or intrarectal route, but it is not limited thereto.

The pharmaceutical composition of the present invention may be administered orally or parenterally and, in the case of parenteral administration, skin application or intraperitoneal, intrarectal, subcutaneous, intravenous, intramuscular or intrathoracic injection method may be selected for administration, but it is not limited thereto.

A preferable administration amount of the pharmaceutical composition may vary depending on the condition and body weight of the affected subject, the severity of disease, the form of the drug, the route and duration of administration, but may be appropriately selected by those skilled in the art. For example, it may be administered so that the number of administered cells per 1 kg of body weight reaches $1 \times 10^3$ to $1 \times 10^9$ cells/kg, preferably $1 \times 10^4$ to $1 \times 10^8$ cells/kg, and more preferably $1 \times 10^5$ to $1 \times 10^7$ cells per 1 kg body weight, but it is not limited thereto. Instead, it may vary and be administered depending on the condition of the patient and the degree of onset of disease. Further, the administration may be once or divided into several times a day. Further, the administration dosage does not limit the scope of the present invention in any way.

The pharmaceutical composition of the present invention may be formulated in a unit dose form using any pharmaceutically acceptable carrier and/or excipient, or may be formulated by introducing the same into a multi-dose container according to any method easily implemented by those skilled in the art, to which the present invention pertains. At this time, the formulation may have the form of a solution, suspension or emulsion in oil or aqueous medium, or the form of extract, powder, granules, tablets or capsules, and may further contain a dispersing agent or stabilizer.

Hereinafter, the present invention will be described in detail and illustrated by means of the following examples.

## EXAMPLE

## Example 1

### (1) Culture of human mesenchymal stem cells (hMSCs)

Adult human mesenchymal stem cells (hMSCs) extracted from normal human bone marrow (Cambrex Bioscience, Walkersville, MD, USA) were cultured in a low-glucose Dulbecco's modified Eagle's medium (DMEM, low glucose) containing 10 % FBS (Gibco, Waltham, MA, USA) added thereto. Cells (passages 6-12) were subjected to passage culture at 37 °C under 5 % $CO_2$ environment for 12 to 15 times thus to obtain late-passage hMSCs.

### (2) Induction of human mesenchymal stem cell into glia-like cell (glial-like cell induced from human mesenchymal stem cell, ghMSC)

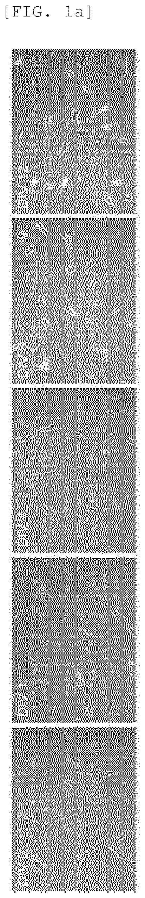

The late-passage hMSCs obtained in Example 1 above were cultured for 24 hours while exchanging the medium with primary-differentiated medium [DMEM-low glucose medium, 10 % FBS, 1 % penicillin-streptomycin mixture, 1 mM β-mercaptoethanol]. Thereafter, the hMSCs were washed with PBS (phosphoric acid buffer solution), and then cultured while exchanging the medium with secondary differentiated medium [DMEM-low glucose medium, 10 % FBS, 1 % penicillin-streptomycin mixture, 0.28 μg/mL all-trans-retinoic acid]. After 3 days, the MSCs were washed with PBS and then cultured for 8 days while exchanging the medium with tertiary differentiated medium [DMEM-low glucose medium, 10 % FBS, 1 % penicillin-streptomycin mixture, 10 μM forskolin, 10 ng/mL human basic-fibroblast growth factor, 5 ng/mL human platelet derived growth factor-AA, 200 ng/mL heregulin-β1]. At this time, the tertiary differentiated medium was exchanged once every other day. For differentiation induction, the form of hMSC under culture was observed by a microscope. The survival of cells during differentiation induction was observed through an optical microscope (FIG. 1a).

### (3) Preparation of concentrated culture solution of glia-like cells

Glia-like cells ($1 \times 10^4$ cells/cm$^2$) were washed with PBS twice, and then cultured for 18 hours with serum-free Neurobasal-A medium (NB medium). After then, the concentrated culture solution was collected and precipitated by means of a centrifuge at 1,500 g for 5 minutes to remove cell waste, followed by using the same in experiments.

### (4) Freezing and thawing of glia-like cells

### 1) Freezing of glia-like cells

1-2 x 10$^5$ hMSCs (or glia-like cells) in DMEM medium containing 10% FBS, FBS crude and DMSO were prepared in a ratio of 5:4:1 to reach 1 mL volume, thereby preparing a freezing vial. Then, the prepared freezing vial was placed in a freezing container containing 100% isopropanol, left in a deep freezer at -80 °C overnight, and then, placed in a liquid nitrogen storage tank (LN2 tank) for storage.

### 2) Thawing of glia-like cell

The freezing vial stored in the liquid nitrogen storage tank was thawed in a water bath at 37 °C for 2 minutes, cells slightly including ice in the freezing vial were well mixed with a pipette in a biosafety hood. 1 mL of cell concentrate in the freezing vial was admixed with 10 mL of DMEM medium containing 10% FBS and placed in 15 mL conical tube. After centrifuging at a speed of 1,200 rpm for 5 minutes, all supernatants were suctioned while remaining the precipitated cell pellets. Then, 1 mL of DMEM medium containing 10% FBS was further added to the 15 mL conical tube and the cell pellets were dispersed with the pipette. 11 μL of cell pellets was admixed with a dye, that is, 11 μL of trypan blue. 10 μL of the mixture was introduced to a cell counter to calculate the number of cells contained in 1 mL of the mixture, followed by spreading the same at 2,000 cells/cm$^2$ on a cell culture dish.

**(5) Analysis of characteristics of glia-like cells induced from late-passage hMSCs**

To confirm the characteristics of glia-like cells derived from hMSCs induced by the method in Example 2, a degree of expression of glia cell marker was determined through immunohistochemical staining.

The cultured cells were fixed with 4% paraformaldehyde (PFA), followed by culturing the cells with 5% standard chlorine serum and 0.1% Triton-X100 blocking solution. The product was stained with GFAP (1:200; Merck millipore), S100 (1:250; Dako) in a refrigerator at 4 °C for 1 day. Then, the stained solution was washed several times with PBS, followed by staining the cells with the secondary antibody, Alexa Fluor®488 anti-mouse IgG (Molecular Probes) and Alexa Fluor®546 anti-rabbit IgG (Molecular Probes) at room temperature for 1 hour. Thereafter, a nucleus was stained with 4,6-diamidino-2-phenylindole (DAPI; Santa Cruz Biotechnology). The sample was observed using a digital inverted fluorescence microscope (DM5000B; Leica).

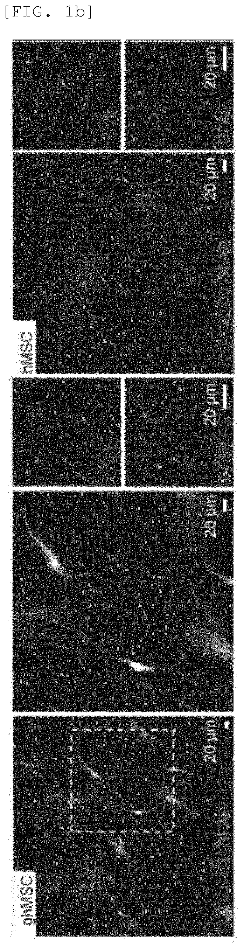

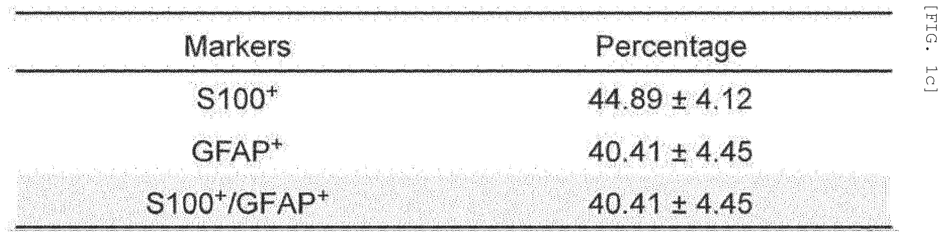

As shown in FIGS. 1b and 1c, it was confirmed that Sox10 and GFAP as the markers of oligodendrocyte were expressed by 45% and 40%, respectively, in the total ghMSCs. These results demonstrate that astrocytes and oligodendrocytes to enhance surrounding environments by facilitating the growth of surrounding cells are included in large quantities in the glia-like cells induced from hMSCs.

**(6) Construction of vascular dementia animal model**

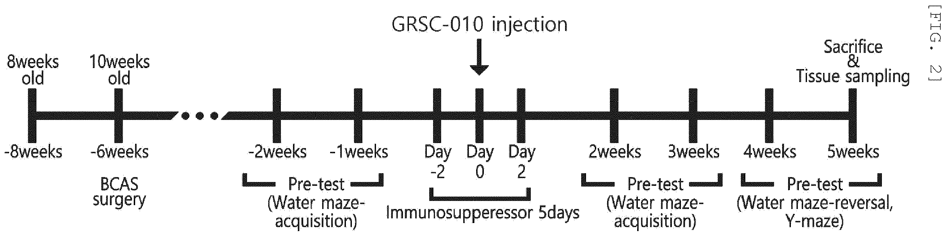

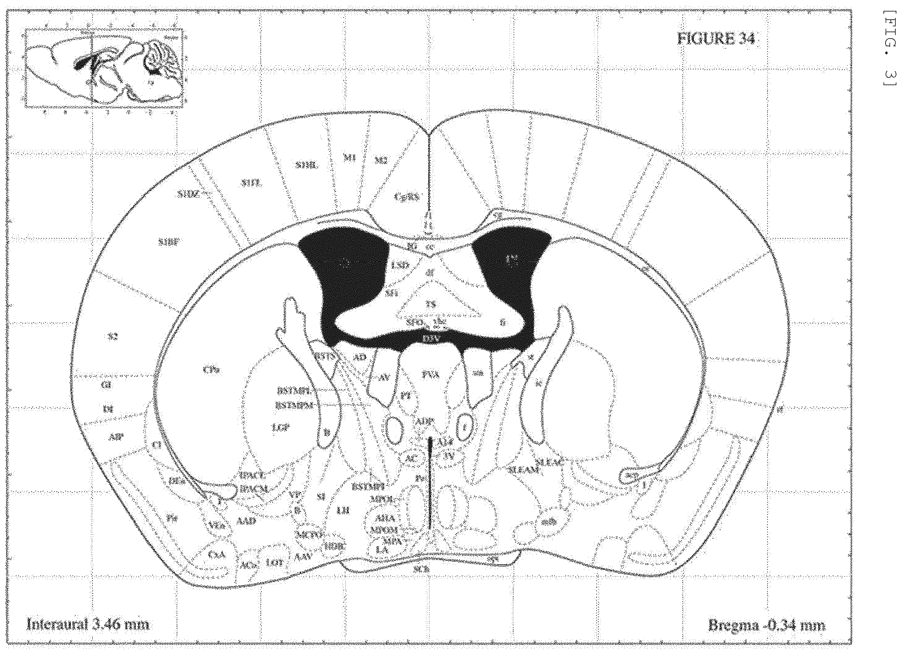

Hereinafter, all animal experiments were executed in compliance with ethical treatment of experimental animal and rules for scientific use (LARL-2022-0017, LARL-2022-0003). Approximate experiment plans for vascular dementia animal model are illustrated in FIG. 2. Specifically, a microcoil having a diameter of 0.18 mm is inserted into both common carotid arteries of 10-week old C57BL/6 mouse and derives global cerebral ischemia due to angiostenosis (FIG. 3). After angiostenosis operation, in order to confirm whether there is any abnormal animal or not, the condition of the animal was visibly monitored once per week. 4 weeks after the operation, in order to confirm whether dementia is derived by chronic decrease n in cerebral blood flow amount, pre-test was executed. As a result of water maze test, a subject having less than 10 probe trials in the obtaining stage was determined not to get dementia.

Further, in order to assess therapeutic efficacy of the glia-like cells in the above vascular dementia animal model, the glia-like cells in the amount of $2 \times 10^5$ cells were introduced into the neurobasal media, followed by targeting bilateral ventricles. Further, $1 \times 10^5$ cells/5 μL of the cells was firstly administered only once to each target at a flow rate of 0.5 μL/min. In the control group, the excipient, that is, 5 μL of neurobasal media was administered in the same manner. Classification of the experimental groups is shown in Table 1 below.

[TABLE 1]

| Group | Total cell number | Amount |
|---|---|---|
| G0 | 0 | 0 μL |
| G1 | 0 | 15 μL |
| G2 | $2 \times 10^5$ | 15 μL |

**(7) Assay of brain tissue by induction of vascular dementia**

5 weeks after the vascular dementia-inducing operation, the experimental group was sacrificed and, after autopsy, the tissue was cold-stored with 4% paraformaldehyde for 2 days or more, and the completely fixed tissue was moved to 30% sucrose solution and cold-stored. After 2 to 3 days, when the brain tissue was settled in 30% sucrose solution, it was moved again to new 30% sucrose solution and then used in the experiment after 24 hours. Thereafter, a frozen section was prepared using the fixed brain tissue. Specifically, only the hippocampus portion was sliced with the coronal plane using a blade, followed by conducting the experiment at -20 °C. In a chuck, a layer of OCT compound was spread and the sliced brain tissue was placed thereon after the compound layer became hard. Then, the brain tissue was scattered over the OCT compound as covering the same as a whole. After waiting the OCT compound to be solidified, a dedicated blade was fitted to a cutter, followed by fitting and fixing the prepared chuck to the same. Then, the brain tissue was cut until a brain tissue with a thickness of 40 μm is obtained and, when the tissue was beginning to come out, the section to be used for experiment was cut into a size of 10 μm. When the sliced section came out, the section was spread out as broad as possible using a brush, and adhered to a slide using a temperature difference by putting the slide thereto. After adhering the desired section to the slide, information was written thereon using a pencil and the slide was placed in a slide box and stored at -18 °C. Thereafter, the corresponding sections were treated with a hematoxylin/eosin solution to stain the brain tissue portion, followed by white matter rarefraction to confirm the degree of lesion.

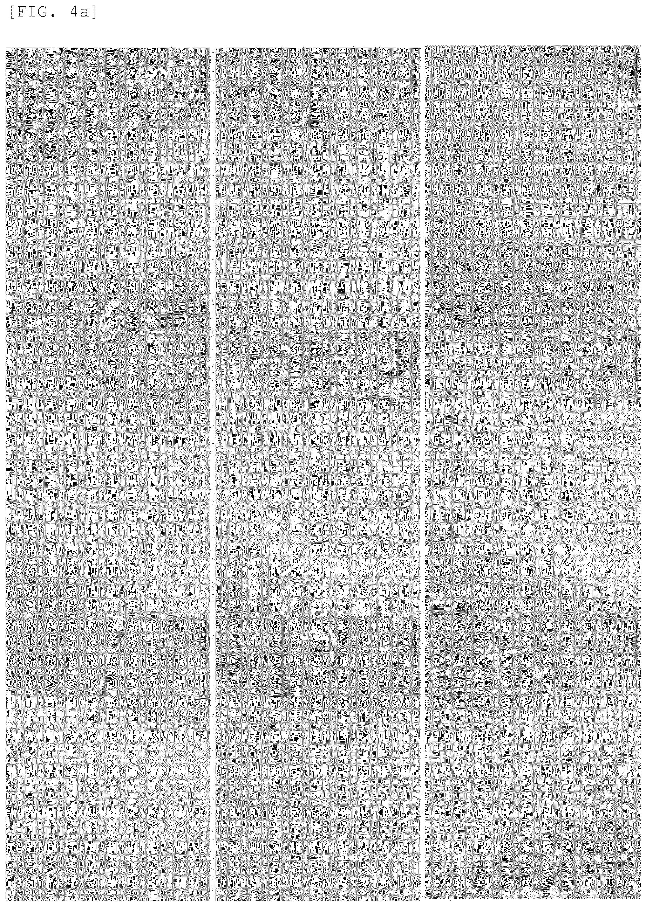

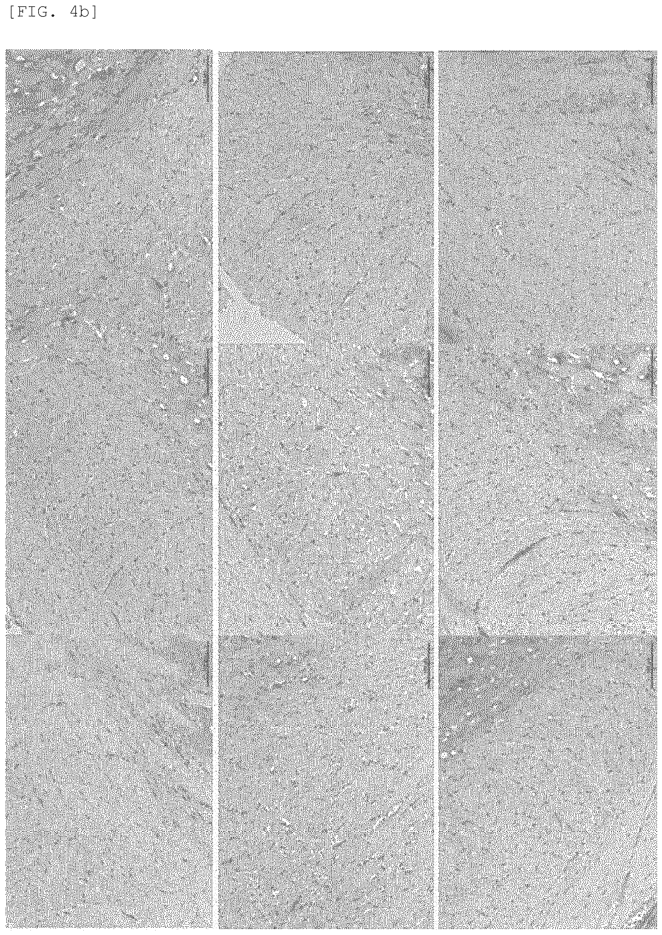

As a result, a group determined to get dementia and another group determined not to get dementia among Coil groups were subjected to staining and observation using a microscope in blind manner. After observation using the microscope, the photographs were divided into groups. Consequently, the groups determined to have dementia, in which coils were treated to both of corpus callosum and internal capsule, showed more significant lesion (see FIGS. 4a and 4b).

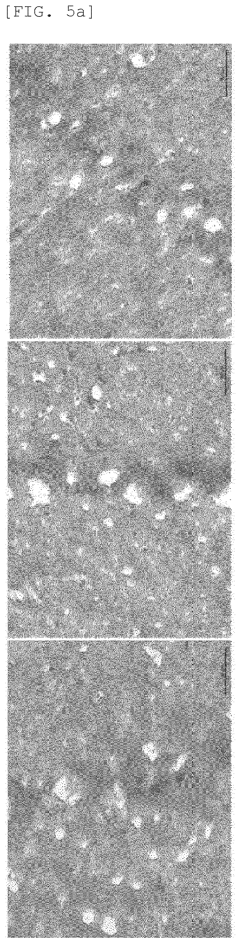

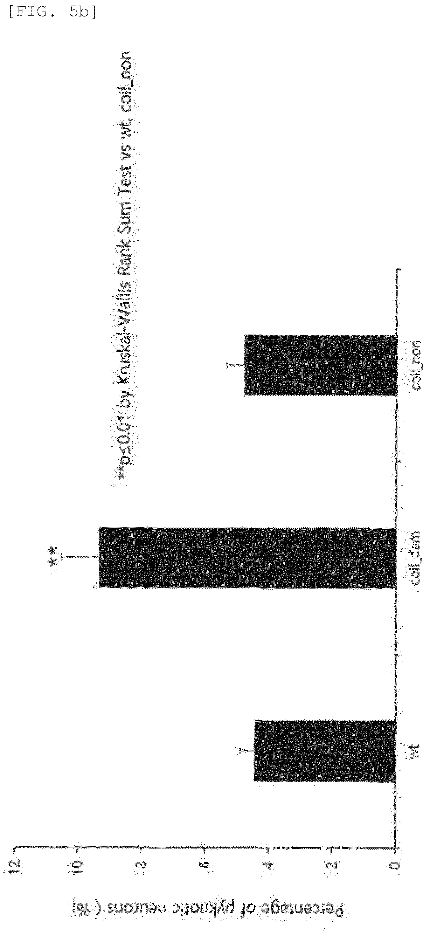

CA1 and CA2 portions of the hippocampus are the most rapidly reacting part when the brain was damaged. According to the reference, a distribution of pyknotic neurons in the corresponding portion was calculated. As a result, the group determined to get dementia, which was subjected to coil operation (9.36±1.2), showed statistically significant difference, as compared to Wild type group (4.45±0.45) and the group determined not to get dementia, which was subjected to coil operation (4.81±0.55) (FIGS. 5a and 5b).

**(8) Water maze test**

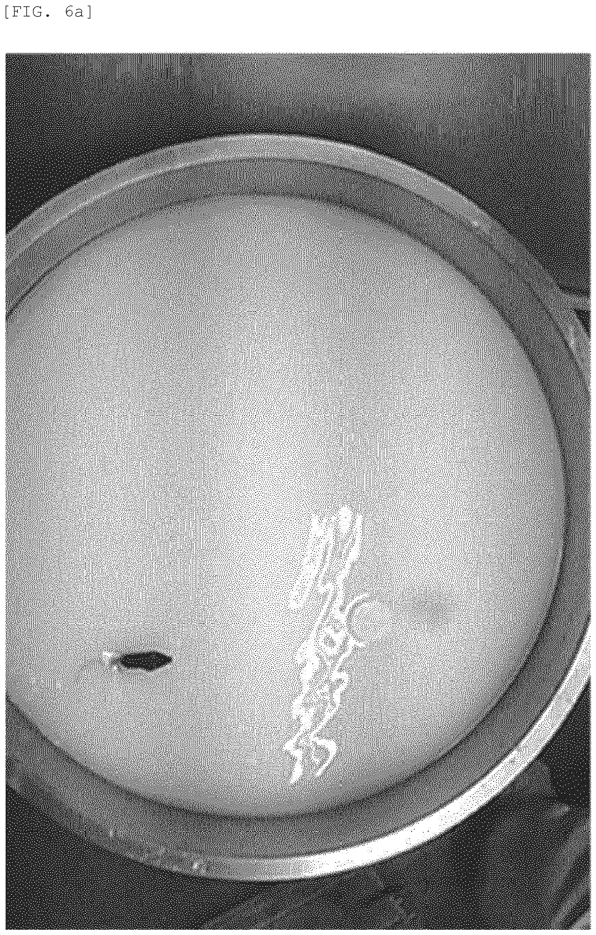

At 2nd week and 4th week after glia-like cell administration, acquisition and reversal stages were performed, respectively. The behavior experimental procedure is the same as shown in FIG. 6a. Specifically, a water bath was filled with water until a platform remains about 2 mm, and then, white dye was added and dissolved thereto. Probe trial was executed in four directions over 5 days and further executed at day 6 in a different direction that had never been attempted before. On each day, a time of finding the platform was recorded and, in the probe trial, the number of times of crossing the platform was also recorded. All experiments were recorded on video using a camera.

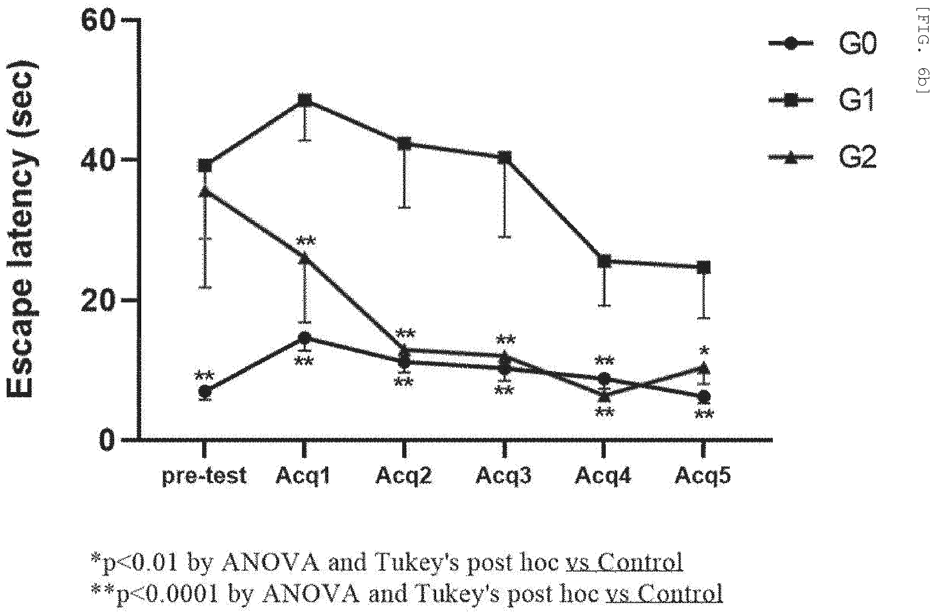

As a result of water maze acquisition, the time of finding the platform in individual groups 14 days after cell administration was confirmed as follows: day 1; G0 (14.42 ± 1.63), G1 (47.18 ± 4.53), G2 (22.56 ± 5.64), day 2: G0 (11.15 ± 1.71), G1 (42.93 ± 4.45), G2 (12.92 ± 1.49), day 3: G0 (10.25 ± 1.11), G1 (40.29 ± 5.37), G2 (12.18 ± 1.52), day 4: G0 (8.8 ± 1.27), G1 (24.14 ± 4.10), G2 (6.42 ± 1.08), day 5: G0 (6.3 ± 0.64), G1 (23.6 ± 4.31), G2 (10.1 ± 2.28). Further, as compared to G1 group, G2 group rapidly found the platform in all days and thus showed statistical significance. Further, G0 group is a normal group and showed statistically significant difference compared to G1 group (P<0.001) (FIG. 6b).

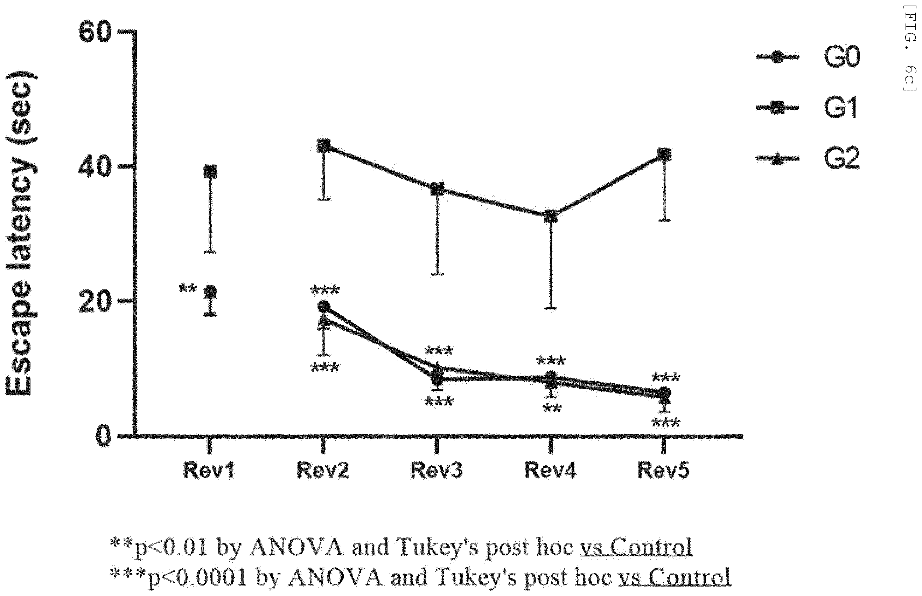

As a result of water maze reversal, the time of finding the platform in individual groups 28 days after cell administration was confirmed as follows: day 1: G0 (21.5 ± 2.91), G1 (40.13 ± 5.48), G2 (22 ± 3.89), day 2: G0 (19.25 ± 3.22), G1 (43.93 ± 4.19), G2 (17.38 ± 3.14), day 3: G0 (8.45 ± 0.96), G1 (34.85 ± 6.32), G2 (10.13 ± 1.54), day 4: G0 (8.89 ± 1.03), G1 (31.38 ± 6.83), G2 (7.71 ± 1.10), day 5: G0 (6.5 ± 0.65), G1 (41.29 ± 4.99), G2 (5.4 ± 1.15). Further, as compared to G1 group, G2 group rapidly found the platform and showed statistical significance from day 2. G0 group is a normal group and showed statistically significant difference compared to G1 group (P<0.01) (FIG. 6c).

**(9) Y maze test**

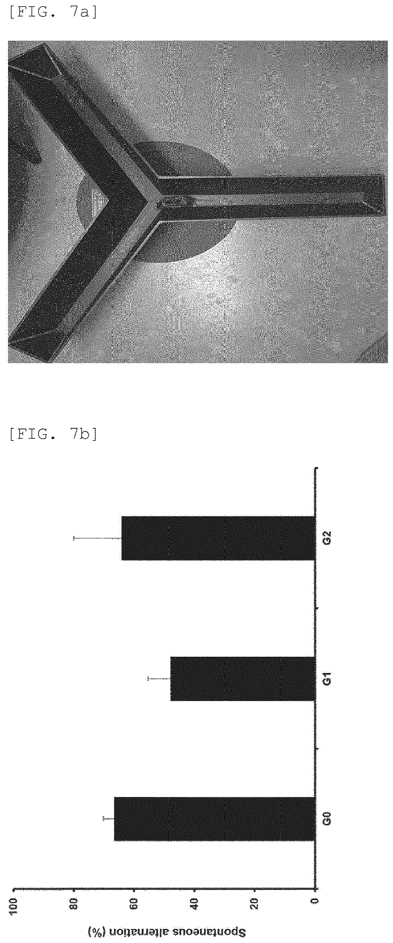

After completing the water maze test on 4th week, Y maze test was executed on 5th week. The behavior experimental procedure is the same as shown in FIG. 7a. Three vertexes of Y maze were set to A, B and C, respectively, all animals started at the same vertex and, when releasing the animals, they were left to go while facing a wall side. Thereafter, the vertexes in which the animal entered were recorded for 5 minutes, followed by calculation using the following Equation 1.

```
[Equation 1]

Spontaneous alternation (%) = [(Number of alternations) /

(Total entries-2)] x 100
```

As a result of Y maze 28 days after cell administration, spontaneous alternations (%), which means making the rounds of the vertexes, in individual groups were confirmed as follows: G0 (66.75 ± 3.36), G1 (47.96 ± 7.32), G2 (64.17 ± 15.91). Further, as compared to G1 group, G2 group had a tendency of higher value but did not show statistical significance (FIG. 7b).

**Example 2**

**(1) Culture of human mesenchymal stem cells (hMSCs)**

hMSC was prepared by the same method as in Example 1 except that passage culture was conducted 9 to 11 times

**(2) Induction of human mesenchymal stem cell into glia-like cell (ghMSC)**

ghMSC was prepared by the same method as in Example 1 except that hMSC of Example 2 was used.

**(3) Assessment of reactivity to transplanted amount of glia-like cells in vascular dementia model**

**1) Configuration of test group and ghMSC administration**

To assess the reactivity to ghMSC transplanted amount in a vascular dementia model, with reference to $1 \times 10^5$ cells/per site, in which the efficacy was confirmed in the previous studies, and the total cell number of $2 \times 10^5$ cells, G2 to G4 groups were set while reducing the number of cells at a geometric ratio of 0.3, and an excipient control group G1 was added thus to divide the groups as shown in the following table (Table 2).

[TABLE 2]

| Group | Total cell number | Amount |
|---|---|---|
| G0 | 0 | 0 $\mu$L |
| G1 | 0 | 10 $\mu$L |
| G2 | $2 \times 10^4$ Cells | 10 $\mu$L |
| G3 | $6 \times 10^4$ cells | 10 $\mu$L |
| G4 | $2 \times 10^5$ cells | 10 $\mu$L |

**2) Water maze**

The experimental method and schedule were performed in the same manner as those of the previous experiments. Water maze was executed to assess a space recognition ability and memory. 1 week before cell administration, only subjects having the probe trial value of 10 seconds or more were determined to get dementia through pre-test, and selected. The control group and cell administered group were randomly assigned and, 2 weeks and 4 weeks after cell administration, individual groups were compared in terms of therapeutic efficacy through water maze.

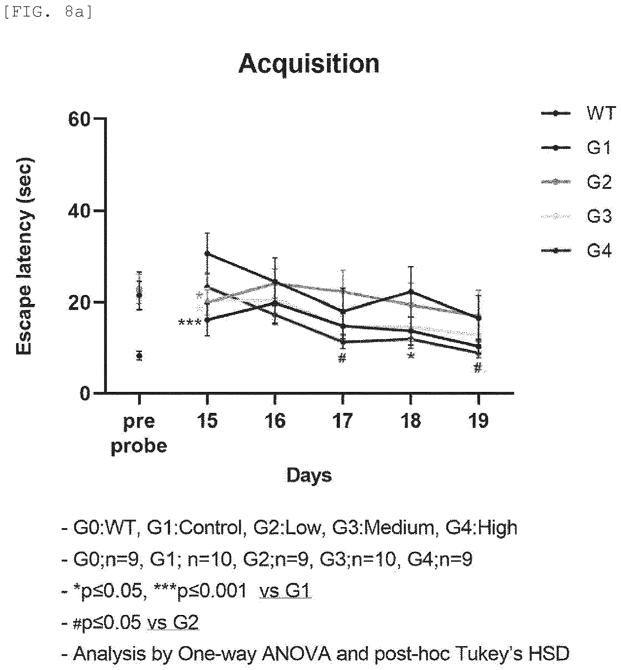

As a result of water maze acquisition 14 days after cell administration, the time of finding the platform in individual groups was confirmed as follows: day 1: G0 (15.75 ± 2.94), G1 (30.60 ± 4.24), G2 (19.87 ± 2.63), G3 (20.21 ± 2.61), G4 (23.25 ± 2.91), day 2: G0 (18.67 ± 3.77), G1 (24.45 ± 4.99), G2 (24.02 ± 2.96), G3 (20.42 ± 4.12), G4 (17.22 ± 1.94), day 3: G0 (14.94 ± 2.35), G1 (18.01 ± 4.74), G2 (22.25 ± 4.49), G3 (14.63 ± 2.27), G4 (11.24 ± 1.37), day 4: G0 (14.44 ± 2.67), G1 (22.23 ± 5.20), G2 (19.34 ± 4.54), G3 (14.58 ± 2.71), G4 (11.90 ± 1.87), day 5: G0 (10.92 ± 1.44), G1 (16.5 ± 4.64), G2 (16.96 ± 5.33), G3 (12.77 ± 3.30), G4 (8.86 ± 1.06). On day 1, G2 group and G3 group showed statistical significance compared to G1 group. However, with the passage of time, it was confirmed that the time of finding the platform became faster with statistical significance for G4 group on day 3 and day 5 compared to G2 group, and on day 4 compared to G1 group, respectively (P<0.05) (FIG. 8a).

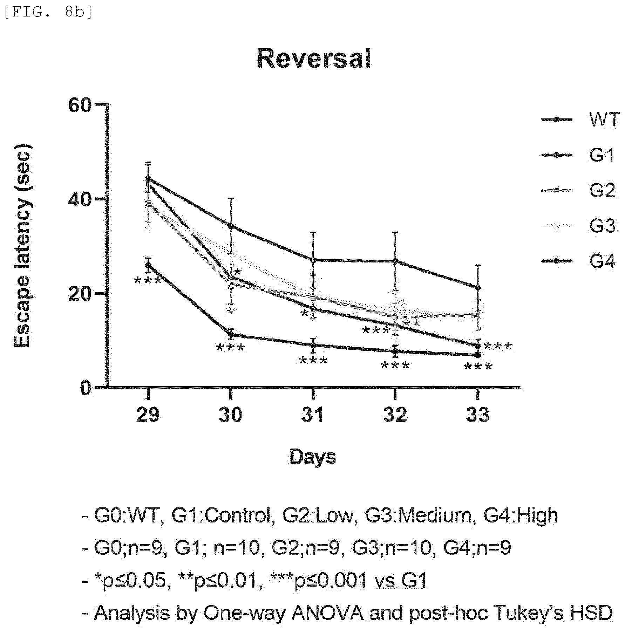

Further, as a result of water maze reversal 28 days after cell administration, the time of finding the platform in individual groups was confirmed as follows: day 1: G0 (15.75 ± 2.94), G1 (30.60 ± 4.24),: G0 (26.61 ± 1.38), G1 (44.37 ± 2.76), G2 (39.30 ± 3.93), G3 (38.13 ± 3.89), G4 (43.22 ± 4.30), day 2: G0 (11.87 ± 1.03), G1 (34.33 ± 5.53), G2 (21.93 ± 3.96), G3 (28.48 ± 1.72), G4 (23.52 ± 2.20), day 3: G0 (10.43 ± 1.86), G1 (27.03 ± 5.66), G2 (19.23 ± 4.40), G3 (19.22 ± 3.11), G4 (16.75 ± 1.79), day 4: G0 (10.25 ± 2.59), G1 (26.83 ± 5.83), G2 (15.06 ± 2.71), G3 (16.36 ± 3.42), G4 (13.28 ± 1.83), day 5: G0 (8.78 ± 1.78), G1 (21.24 ± 4.58), G2 (15.49 ± 3.04), G3 (14.98 ± 2.30), G4 (8.81 ± 1.32). Further, as compared to G1 group, G4 group rapidly found the platform and showed statistical significance from day 2 (p<0.05). Further, as compared to G1 group, G2 group showed statistical significance on day 2 and day 4, while G3 group showed statistical significance (p<0.05) (FIG. 8b).

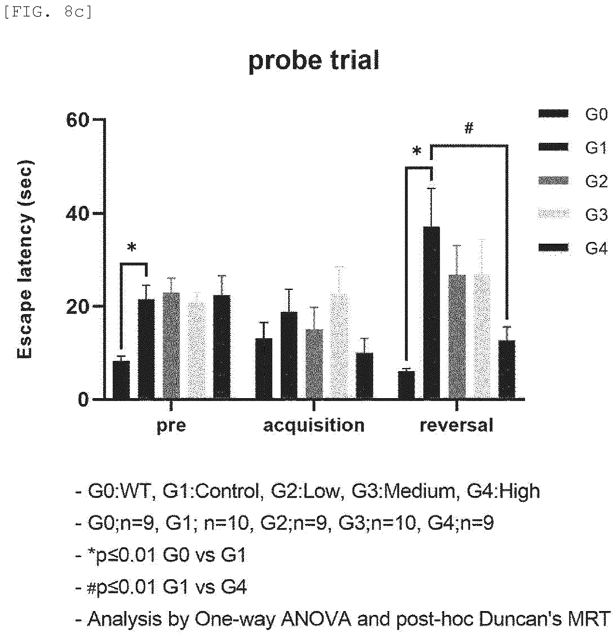

The probe trial in which the animal is placed in a direction never attempted before in the water maze stage and was performed for individual groups, and the results in the pre-test were confirmed as follows: G0 (8.22 ± 0.86), G1 (21.5 ± 2.88), G2 (22.89 ± 3.02), G3 (20.80 ± 2.19), G4 (22.44 ± 3.90), acquisition: G0 (12.11 ± 3.08), G1 (18.80 ± 4.65), G2 (15.11 ± 4.43), G3 (22.50 ± 5.74), G4 (10 ± 2.95), reversal: G0 (7.33 ± 1.23), G1 (37.10 ± 7.78), G2 (26.78 ± 5.98), G3 (27.00 ± 7.17), G4 (12.67 ± 2.84). Before cell administration, it was confirmed that the test groups except for the normal group G0 did not have significant difference. On the other hand, G0 group showed statistically significant difference compared to all other groups ($p \leq 0.01$). Further, 2nd week after cell administration, there was no statistical significance in the acquisition stage, however, G2 group and G4 group were confirmed to have a tendency of quickly finding the platform compared to G1 group. Further, in the reversal stage on 4th week after administration, G4 group showed statistically significant difference compared to G1 group ($p \leq 0.01$), while G0 group as the normal group also showed difference compared to G1 group ($p \leq 0.01$). Further, G2 group and G3 group did not show statistical significance, however, showed a tendency of reduced time for fining the platform compared to G1 group (FIG. 8c).

### Example 3

**(1) Comparison experiment of transplantation of mesenchymal stem cells before differentiation and glia-like cells in vascular dementia model**

**1) Configuration of test group and cell administration**

Based on the ghMSC amount of $1 \times 10^5$ cells/per site, at which therapeutic efficacy was confirmed in the vascular dementia animal model, it was intended to compare effects thereof by administering hMSCs in the stage before differentiation. The hMSC group G2 to which the same concentration of cells was transplanted, the ghMSC group G3, the excipient control group G1, and the normal control group G0 were prepared and divided as shown in the following table (Table 3).

[TABLE 3]

| Group | Total cell number | Amount |
|---|---|---|
| G0 | 0 | 0 μL |
| G1 | 0 | 10 μL |
| G2 | $2 \times 10^5$ cells | 10 μL |
| G3 | $2 \times 10^5$ cells | 10 μL |

**2) Water maze**

The experimental method and schedule were performed in the same manner as those of the previous experiments. Water maze was executed to assess a space recognition ability and memory. Further, the control group and the cell administered group were randomly assigned based on the pre-probe trial value. 2 weeks and 4 weeks after cell administration, individual groups were compared in terms of therapeutic efficacy through water maze.

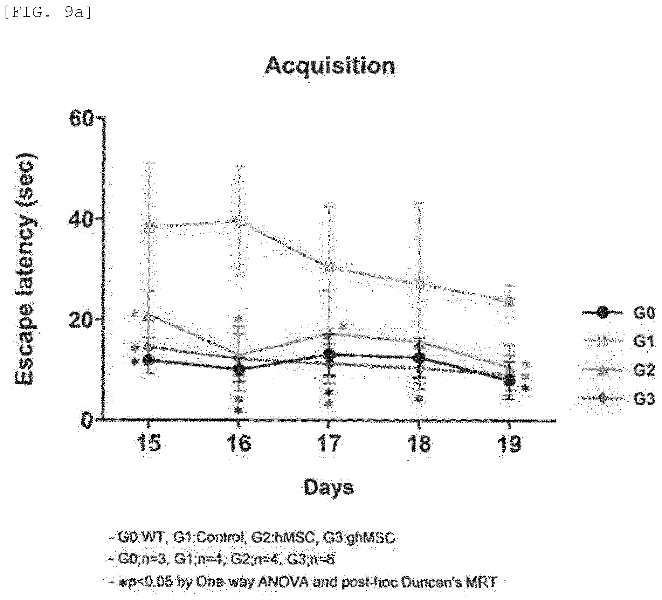

As a result of water maze acquisition 14 days after cell administration, the time of finding the platform in individual groups was confirmed as follows: day 1: G0 (11.92 ± 0.25), G1 (38.40 ± 5.50), G2 (21 ± 2.00), G3 (14.54 ± 1.96), day 2: G0 (10.08 ± 1.13), G1 (39.60 ± 4.71), G2 (12.94 ± 1.76), G3 (12.21 ± 2.37), day 3: G0 (13.08 ± 1.94), G1 (30.38 ± 5.25), G2 (17.21 ± 3.74), G3 (11.29 ± 1.46), day 4: G0 (12.5 ± 1.85), G1 (27.13 ± 7.01), G2 (15.69 ± 3.53), G3 (10.38 ± 1.51), day 5: G0 (8.08 ± 1.73), G1 (23.83 ± 1.36), G2 (10.56 ± 1.96), G3 (9.13 ± 1.48). As compared to G1 group, the time of finding the platform was statistically and significantly reduced in both of G2 group and G3 group over all days ($p<0.05$). However, G3 group only showed statistical significance ($p<0.05$) on day 18. Further, in terms of the finding time, G3 group has found the platform in the time closer to or faster than G0 group as the normal group, therefore, it is predicted that G3 group has more effective efficacy (FIG. 9a).

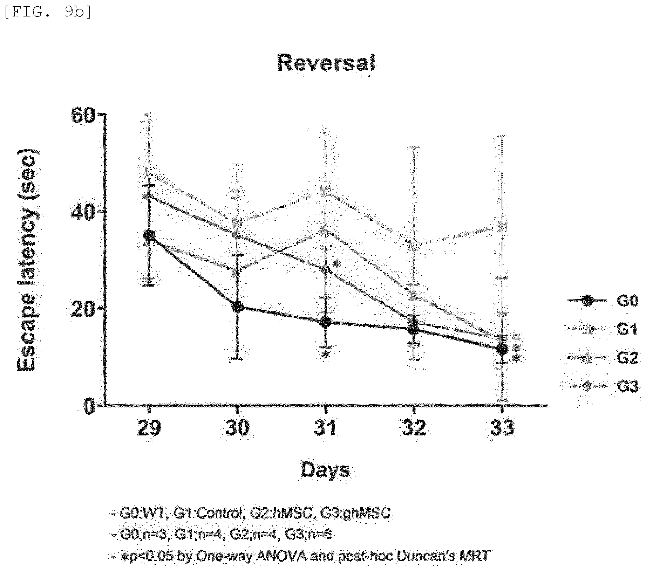

Further, as a result of water maze reversal 28 days after cell administration, the time of finding the platform in individual groups was confirmed as follows: day 1: G0 (35.08 ± 4.84), G1 (48.02 ± 5.22), G2 (34.08 ± 3.73), G3 (43.07 ± 6.30), day 2: G0 (20.33 ± 5.05), G1 (37.60 ± 5.25), G2 (27.75 ± 7.11), G3 (35.17 ± 2.84), day 3: G0 (17.17 ± 2.43), G1 (44.23 ± 5.18), G2 (36.33 ± 1.48), G3 (27.97 ± 3.26), day 4: G0 (15.67 ± 1.37), G1 (33.06 ± 8.74), G2 (22.81 ± 4.54), G3 (17.22 ± 2.87), day 5: G0 (11.58 ± 1.34), G1 (37 ± 7.99), G2 (13.21 ± 2.52), G3 (13.67 ± 4.70). The platform finding time was significantly reduced in G3 group on day 31, and in G2 group and G3 group on day 33 ($p<0.05$) (FIG. 9b).

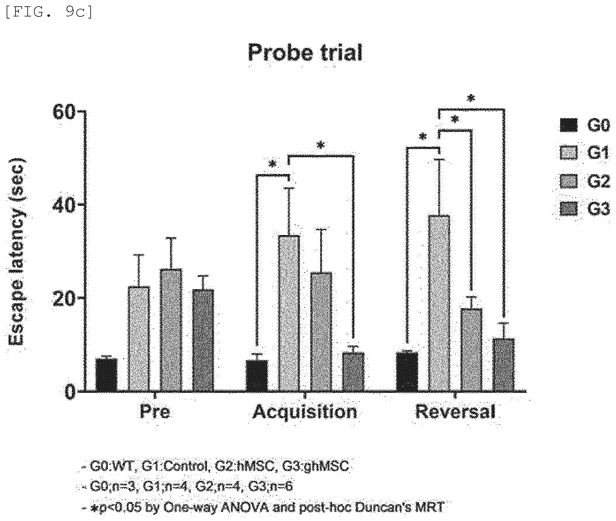

The probe trial in which the animal is placed in a direction never attempted before in the water maze stage and was performed for individual groups, and the results in the pre-test were confirmed as follows: G0 (7 ± 0.47), G1 (22.5 ± 5.86), G2 (26.25 ± 5.73), G3 (21.83 ± 2.67), acquisition: G0 (6.67 ± 1.09), G1 (33.5 ± 8.69), G2 (25.5 ± 7.95), G3 (8.33 ± 1.19), reversal: G0 (8.33 ± 0.27), G1 (37.75 ± 10.35), G2 (17.75 ± 2.16), G3 (11.33 ± 3.03). Before cell administration, it was confirmed that the test groups except for the normal group G0 did not have significant difference. But, 2nd week after cell administration, it could be confirmed that G3 group only quickly found the platform with statistic difference in the acquisition stage, compared to G1 group (p<0.05). Further, in the reversal stage on 4th week after administration, both of G2 group and G4 group showed statistically significant difference compared to G1 group (p≤0.05), however, it could be confirmed that the finding time was shorter in G3 group than G2 group. Further, G0 group as the normal group also showed difference compared to G1 group (p<0.05) (FIG. 9c).

**(2) VEFG-A, IGFBP-4 ELISA**

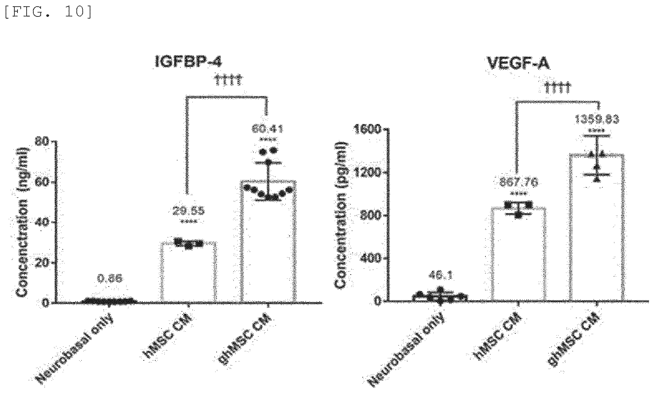

The undifferentiated hMSC and the ghMSC after completion of differentiation were cultured in Neurobasl medium for 18 hours. Thereafter, using RayBio® Human VEGF-A ELISA Kit (For Lysates), R&D Systems Co., DuoSet human IGFBP-4 ELISA kit, a concentration of VEGF-A protein in hMSC-CM and ghMSC-CM was determined according the instruction described in the manual.
Referring to FIG. 10, it could be confirmed that the concentrations of VEGF-A and IGFBP-4 in ghMSC-CM were remarkably increased.

**(3) Flow cytometry**

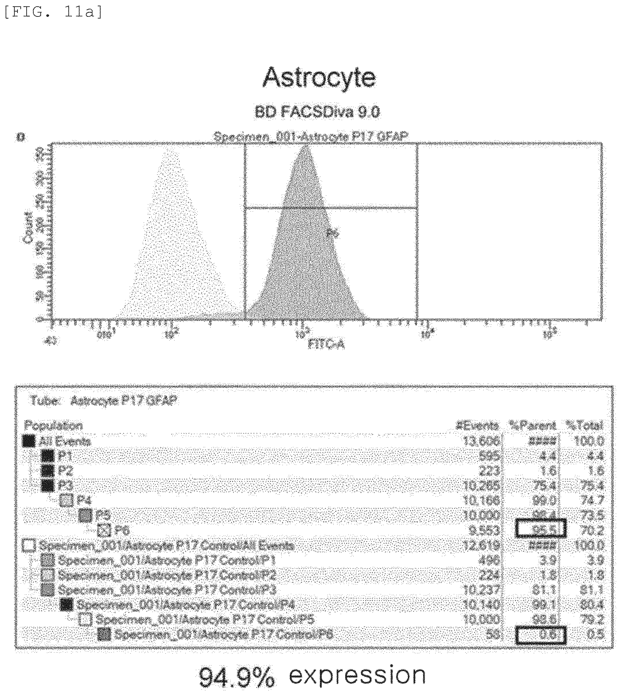

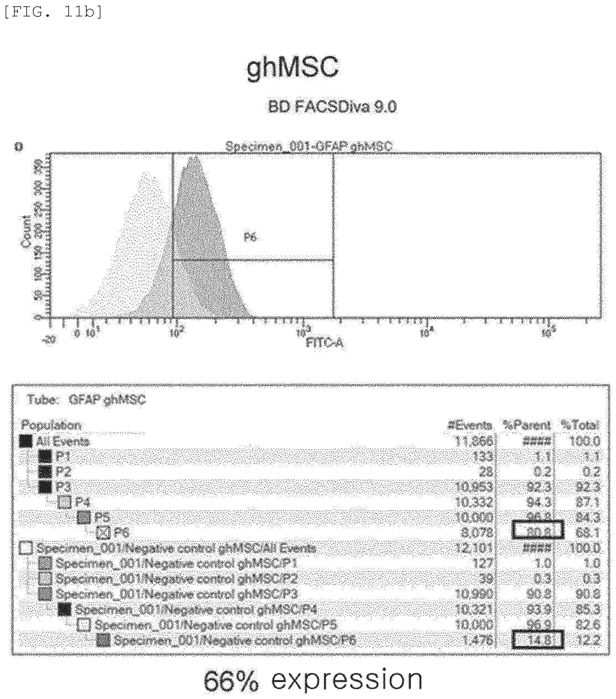

After fixing the above cells with 4% paraformaldehyde (PFA) for 20 minutes, the cells were washed twice with FACS buffer (5% FBS/PBS). Then, for permeation of an antibody, the cells were treated with 0.4% Triton X-100 in PBS, followed by washing twice with FACS buffer (5% FBS/PBS). A primary antibody (GFAP) was adhered to the cells, followed by reaction at 4 °C for 1 hour. After washing the product twice with FACS buffer (5% FBS/PBS), it was subjected to assay in a flow cytometry device.
Referring to FIG. 11, it could be confirmed that the expression of GFAP protein was increased in ghMSC induced from hMSC.

**(4) Cytokine array**

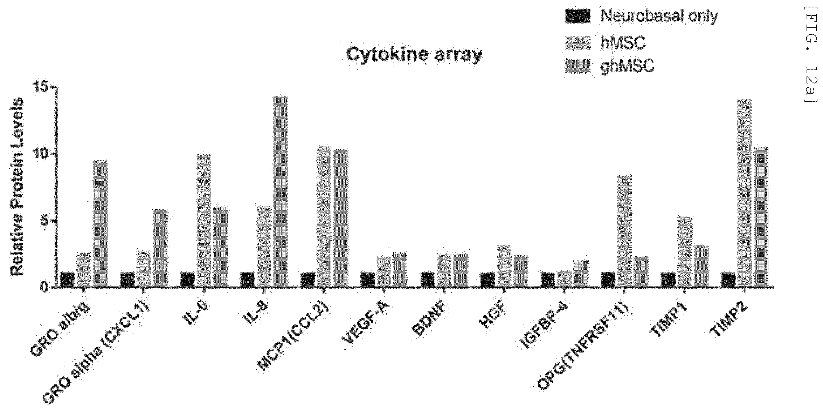

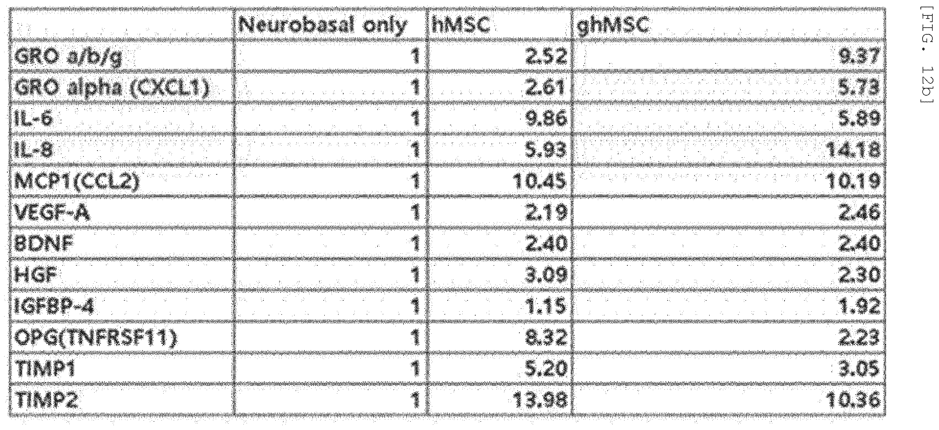

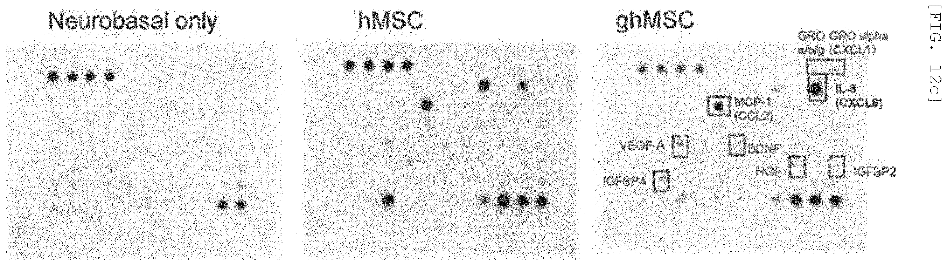

Undifferentiated hMSC and differentiation-terminated ghMSC were cultured for 18 hours in Neurobasal media. Then, according to RayBio® Human Cytokine Antibody Array C5 kit manual method, the tendency of cytokine expression in each of the neurobasal media, hMSC-CM and ghMSC-CM was investigated.
Referring to FIG. 12, it could be confirmed that the expression of cytokine such as GRO a/b/g, GRO alpha (CXCL1), IL-8, etc. in ghMSC-CM was increased, as compared to neurobasal only and hMSC-CM,

**(5) Confirmation of ghMSC-CM protection effects in mouse brain endothelial cells (bEnd.3 cells)**

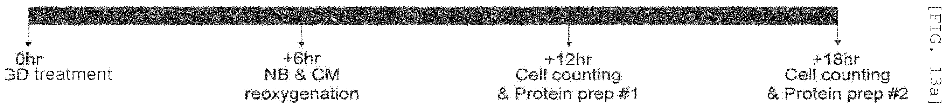

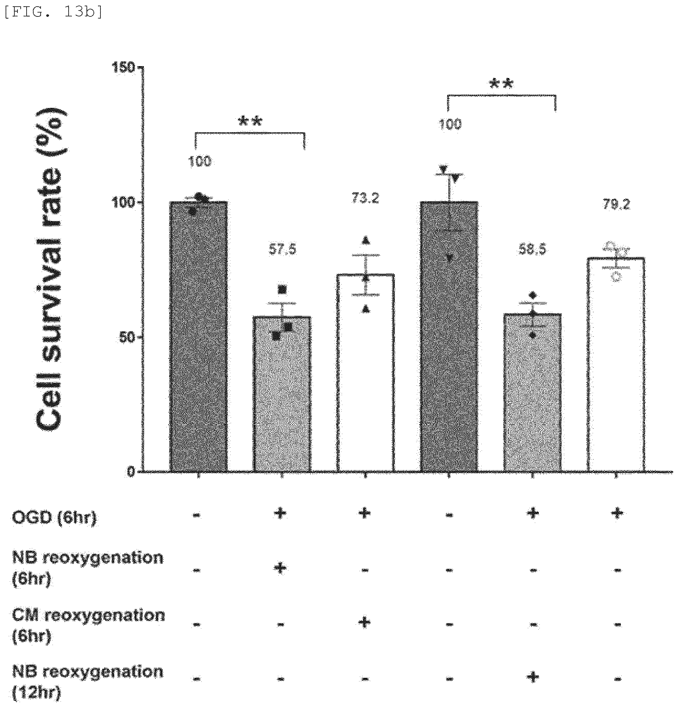

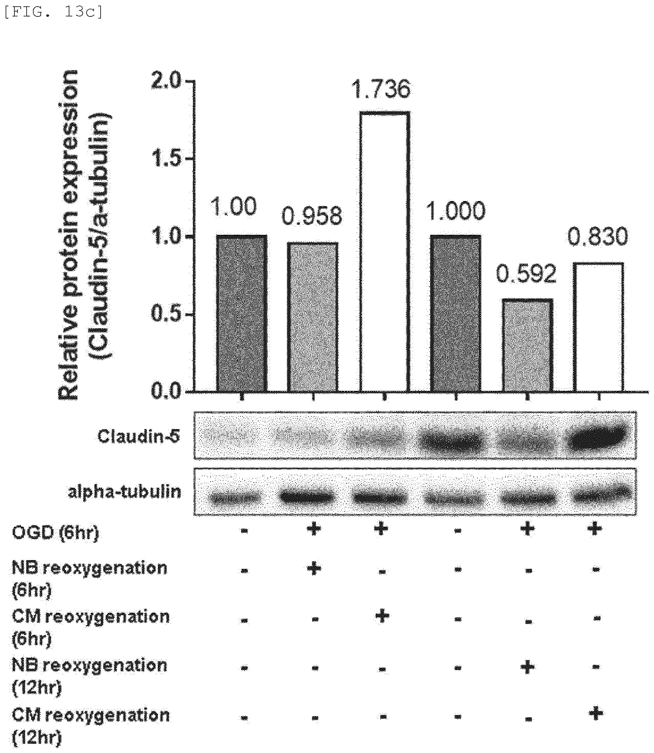

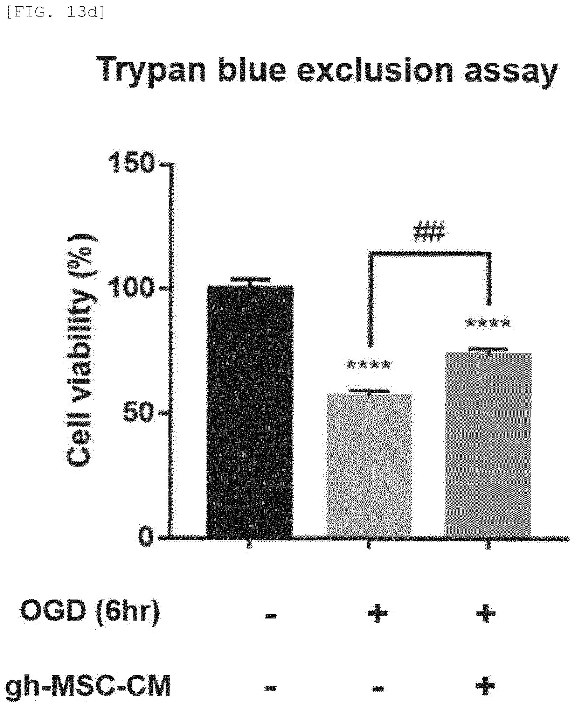

After culturing bEnd.3 cells to reach 70 to 80% confluency, the cells were treated with a mixed gas consisting of 95% $N_2$ and 5% $O_2$ and glucose-free DMEM to establish an oxygen-glucose deprivation environment. After OGM treatment for 6 hours, reoxygenation was performed with regard to the existing growth media, that is, high glucose DMEM as the control group, the neurobasal media only as the OGD-only group, and ghMCS-CM as the ghMSC-CM group, respectively. After conducting reoxygenation for 6 hours and 12 hours, respectively, cell survival rate was compared between the groups through cell counting.
The cell survival rate when reoxygenation was performed for 6 hours and 12 hours was reduced to 57% and 58%, respectively, in the neurobasal only media, as compared to the control group. On the other hand, when the reoxygenation was performed for 6 hours and 12 hours, respectively, in ghMSC-CM, the cell survival rate was determined to reach about 73% and 79%, respectively (FIG. 13b). Through these results, it was confirmed that ghMSC-CM could contribute to the survival rate of bEnd.3 cells (One way ANOVA Test, Control vs. OGD : **P < 0.01).
After conducting OGD for 6 hours, the cells were treated with each of the neurobasal only media and ghMSC-CM for reoxygenation for 6 hours and 12 hours, respectively, followed by investigating the expression of Claudin-5 protein through Western blotting. When NB reoxygenation was performed for 12 hours after OGD treatment, the protein expression was reduced to about 0.59 times. On the other hand, when ghMSC-CM reoxygenation was performed for 12 hours, it was observed that the protein expression was recovered to about 0.83 times (FIG. 13c). Through these results, it was confirmed that ghMSC-CM could contribute to the recovery of claudin-5 protein expression in bEnd.3 cells. Claudin-5 is a tight junction marker of blood-brain barrier.
After conducting OGD ($N_2$ 95%, $CO_2$ 5%) for 6 hours, bEnd.3 cells were treated with each of the neurobasal media (NB) and ghMSC-CM for 12 hours, respectively, followed by determining the cell survival rate through trypan blue exclusion assay. It was observed that the cell survival rate was reduced to about 57% in the NB treated cell group compared to the control group, while the cell survival rate after ghMSC-CM treatment was increased to about 73% (FIG. 13d). Through these results, it was confirmed that ghMSC-CM could contribute to the recovery of bEnd.3 cell survival rate (One way ANOVA Test, Control vs : ****$P < 0.0001$, OGD vs CM ##$P < 0.01$).

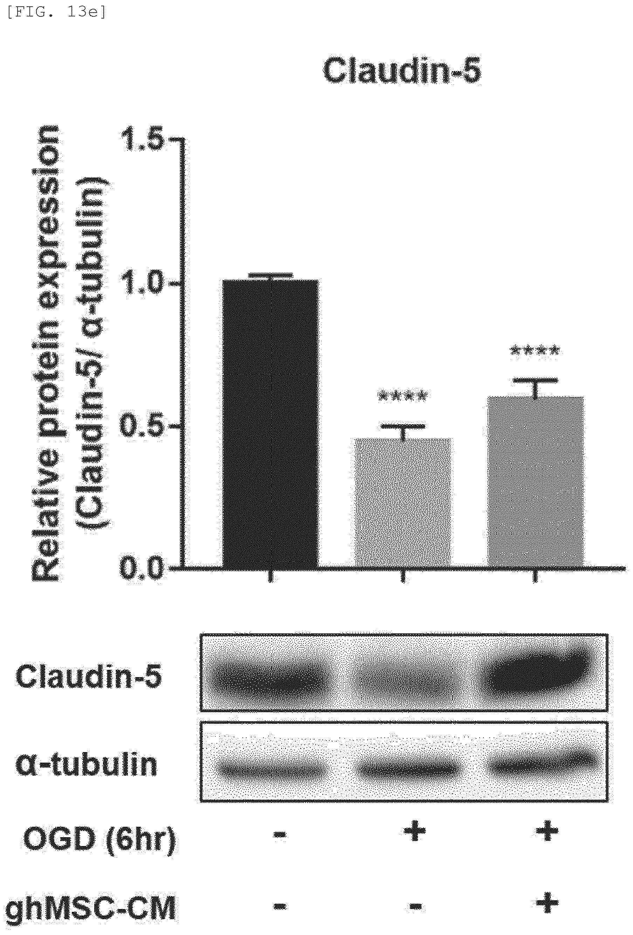

After conducting OGD ($N_2$ 95%, $CO_2$ 5%) for 6 hours, bEnd.3 cells were treated with each of NBa and ghMSC-CM for 12 hours, respectively, followed by investigating the relative expression of Claudin-5 protein through Western blotting. It was observed that the cell group subjected to NB treatment after completing OGD treatment has Claudin-5 expression decreased to about 45%, whereas the cell group subjected to ghMSC-CM treatment showed the expression increased to 60% (FIG. 13e). Through this, it was confirmed that ghMSC-CM could contribute to the expression of claudin-5 protein in bEnd.3 cells (One way ANOVA Test, Control vs : ****$P < 0.0001$, OGD vs CM).

### (6) Organotypic brain (Hippocampus) slice culture

From P7 (Postnatal 7, 7 days after birth) rat brain, the hippocampus was separated and sliced into a thickness of 350 $\mu$m using a chopper.

A medium prepared by adding MEM + HEPES mixture (50%), horse serum (25%), Hank's solution (25%), 6.5 mg/ml glucose and 1% Penicillin/Streptomycin was used. After culturing for 4 days, the product was subjected to a reaction for 2 hours using 5 ug/mL of propidium iodide (PI) before OGD (95% $N_2$, 5% $CO_2$ gas) treatment, followed by observation using a fluorescence microscope (F0).

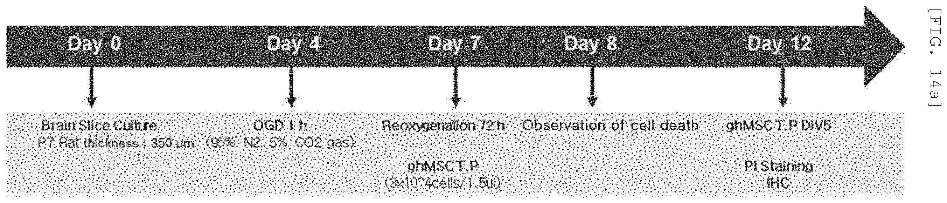

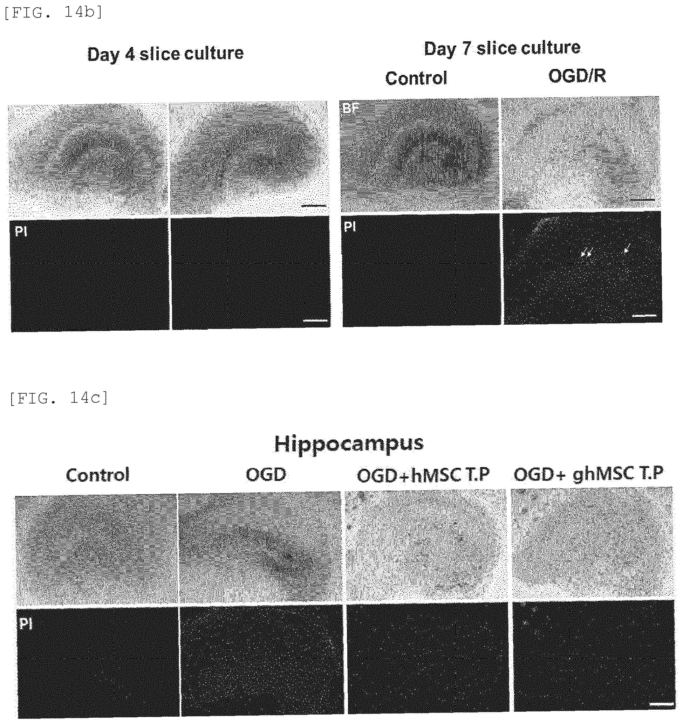

FIG. 14a is an experimental schematic view and FIG. 14b illustrates results of the experiment.

In FIG. 14b, A is an image 4 days after culturing the hippocampus slice. Through PI staining (5 ug/ml, 2 hours), it was confirmed that there is no damage in tissues. Then, the sample was divided into the control group and the OGD group, followed by performing OGD experiment.

In FIG. 14b, B is a cell death image 3 days after reoxygenation following deprivation of oxygen and glucose. When PI staining was performed in the tissue of the control group, low cell death was confirmed. Further, cell death was also confirmed in the OGD/R group (OGD1h, Reoxygenation 72h), and the OGD-induced hippocampus injury model was established (white arrow indicates cell death stained with PI).

### (7) Oxygen-glucose deprivation (OGD) induced rat brain (Hippocampus) injury model

By bubbling the OGD solution into 95% $N_2$ gas and 5% $CO_2$ gas, oxygen was removed. After culturing the hippocampal slice for 4 days, it contacted the OGD solution. After injecting 95% $N_2$ gas and 5% $CO_2$ gas to an air-tight bag chamber, the culturing was performed in an incubator at 37 °C for 1 hour. After 1 hour, the product was washed and then subjected to reoxygenation using a serum free slice culture medium for 72 hours.

After completing the reoxygenation for 72 hours, the product was subjected to PI solution (PI 5 ug/ml + Serum free culture media) treatment, followed by observing whether there is cell death through a fluorescence microscope (Ft). For assessment of the cell death, the medium was replaced with PBS, the tissue was stored at 4 °C for 24 hours, and then PI staining was again conducted, followed by observation using a fluorescence microscope (F fin). Image assessment was performed by setting 8-bit through Image J, measuring a gray-value mean intensity, and then evaluating a percentage of cell death through Equation 2 below.

[Equation 2]

$$\% \text{ Cell death evaluation formula } = (Ft-F0)/(Ffin-F0) \times 100$$

(F0 : Basal fluorescence intensity, Ft : OGD/Reoxygenation fluorescence intensity, Ffin : Cell all kills fluorescence intensity)

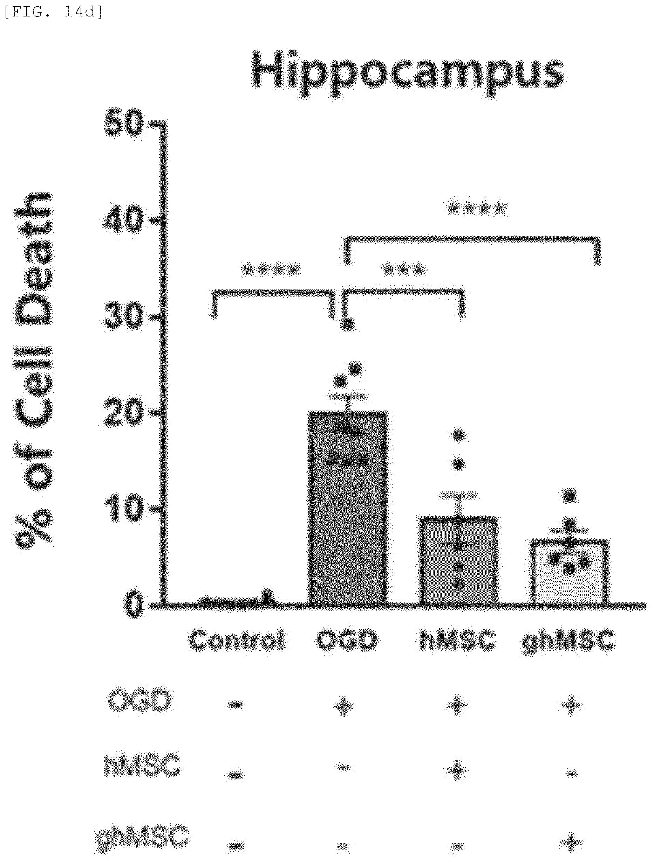

After culturing the hippocampal slice obtained from P7 rat for 4 days, oxygen-glucose deprivation (OGD) was performed for 1 hour, followed by investigating whether there is cell death or not. On day 3 after performing OGD/Reoxygenation, hMSC and ghMSC were transplanted. Then, after 5 days, whether there is cell death or not was evaluated (FIG. 14c and d).

When comparing the OGD group to the control group, the OGD group showed about 20% cell death rate. Further, hMSC and ghMSC showed decrease in cell death rate of 9% and 6.6%, respectively, as compared to the OGD group (One way ANOVA test, Control vs OGD; **** $P < 0.0001$, OGD vs hMSC, OGD vs ghMSC *** $P < 0.001$, **** $P < 0.0001$).

**(8) Assessment of ghMSC and hMSC *in vivo* transplantation effects in OGD-induced hippocampus injury model**

3 days after performing OGD (Reoxygenation 72h), ghMSC and hMSC transplantation in tissue was performed, Using a microcapillary tip, dissociation was performed for the ghMSC with Tryple Select and the hMSC with Trypsin, followed by cell counting. Then, after spinning down each product at 2000 g for 3 minutes, tissue transplantation was performed at $3 \times 10^4$ cells/1.5 $\mu$L/slice. Thereafter, the product was subjected to PI staining and fixing on day 5.

5 days after OGD performing, Control, OGD, OGD+hMSC and OGD+ghMSC tissues, respectively, were washed with PBS and fixed with 4% PFA. On the next day, the fixed sample was washed with PBS three time by 10 minutes each time.

After permeabilization with 0.5% Triton X-100 (1% BSA in PBST) for 20 minutes, the product was subjected to blocking with 3% BSA (0.1% TX-100 in PBS) while gently stirring for 1 hour. To the blocked product, a primary antibody MAP2 (Rabbit, 1:400) and hNu (Mouse, 1:200) was bound to the tissue at 4 °C. On the next day, the treated product was washed with PBS, then secondary antibodies Alexa 48 rabbit, 1:200 and Alexa 546 mouse 1:200 were bound for 1 hour, followed by reacting the product with 0.1 $\mu$g/mL DAPI while shielding light. After washing with PBS, the product was sealed on a slide and observed using LEICA fluorescence microscope.

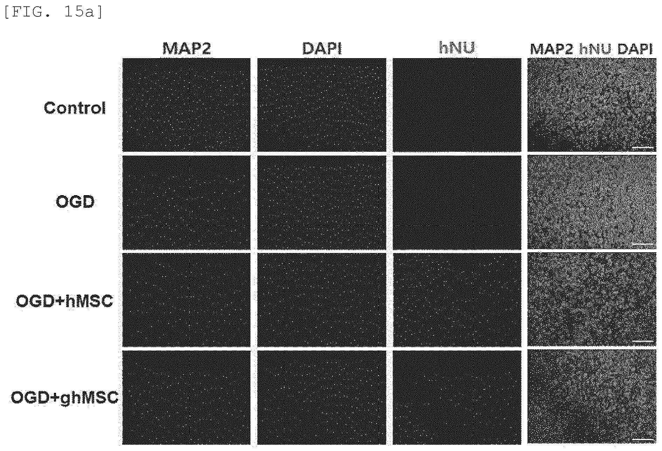

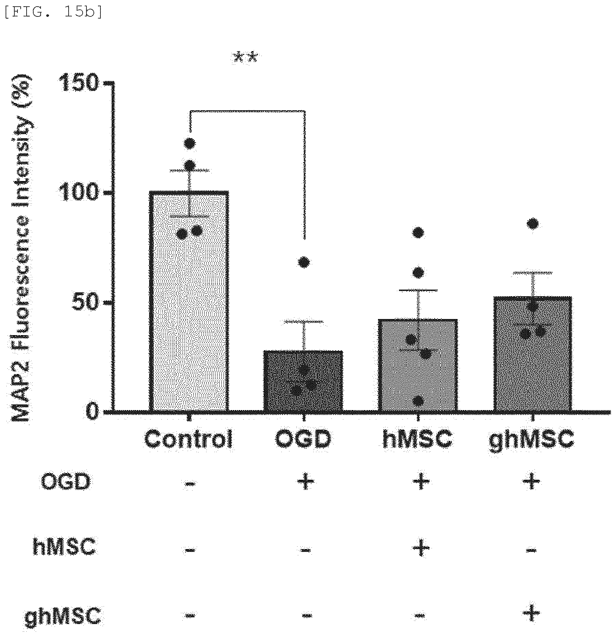

On day 3 after performing OGD/Reoxygenation, hMSC and ghMSC were transplanted. After 5 days, according to MAP2 (microtubule associated protein 2, neuronal marker) staining, cell death and ghMSC T.P. effects in the injury model were evaluated (FIGS. 15a and b). As a result of comparing the OGD group to the control group, the OGD group showed a fluorescent intensity of about 27.7% for MAP2. Further, hMSC and ghMSC showed increase in fluorescent intensity of MAP2 by 42.3% and 51.9%, respectively, as compared to the OGD group (One way ANOVA test by Tukey, ** P < 0.05 vs Control).

**(9) qRT-PCR results of glia-like cells derived from human mesenchymal stem cells**

RNA was extracted using TRIzol reagent (Invitrogen, Carlsbad, CA, USA), and then treated with DNase. For cDNA synthesis, reverse transcription was performed using M-MLV reverse transcriptase (Promega) at 42 °C for 1 hour. Using SYBR FAST qPCR Kits (KAPA Biosystems), genes expressed in cDNA were confirmed with gene-specific primers having SEQ ID NOs: 1 to 24, respectively. The degree of expression of a target gene was confirmed with reference to GAPDH using Ct method. $\triangle$Ct value is a vale obtained by subtracting Ct value, while a cycle number of critical value was measured as ($\triangle$Ct=Ct(Target)-Ct(GAPDH)). A relative value of target gene to endogenous GAPDH was determined by fold-change of GAPDH=$2^{-\triangle Ct}$.

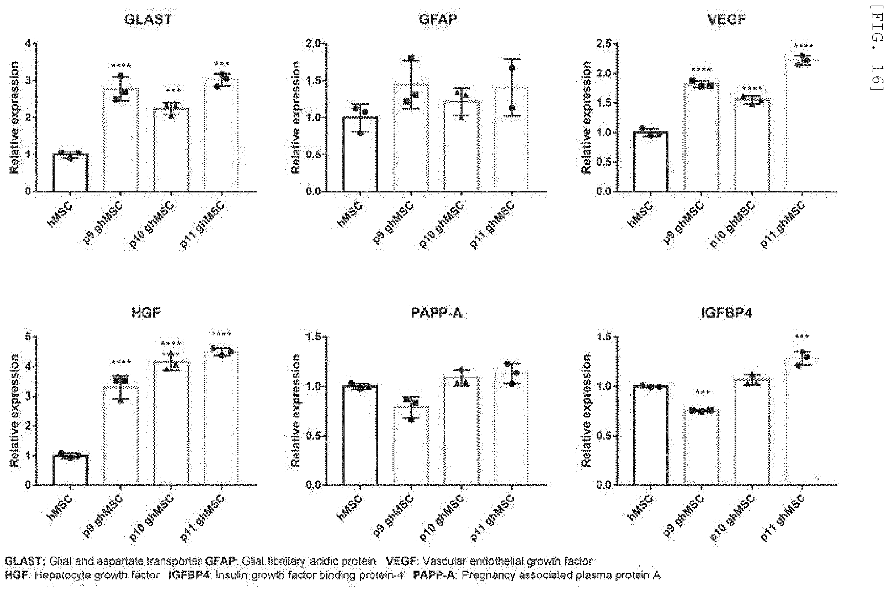

Through real-time qPCR, a differentiated marker of ghMSC was quantitatively determined in mRNA level, and the ghMSC was confirmed to show a tendency of expression in that the differentiated marker is increased without a difference according to passage, as compared to undifferentiated hMSC (FIG. 16) .

Differentiation marker: VEGF, GFAP, HGF, GLAST, IGFBP-4, PAPP-A.

(One way ANOVA test, ***P < 0.001,****P < 0.0001 vs. hMSC)

**Claims**

1. A pharmaceutical composition for prevention or treatment of vascular dementia, comprising glia-like cells differentiated from human mesenchymal stem cells (hMSCs).

2. The pharmaceutical composition according to claim 1, wherein the glia-like cell expresses one or more marker gene selected from the group consisting of GFAP, GLAST, HGF, VEGF, IGFBP-4 and PAPP-A.

3. The pharmaceutical composition according to claim 1, wherein the human mesenchymal stem cell is derived from tissues selected from the group including bone marrow, fat, peripheral blood, cord blood, muscle and skin.

4. The pharmaceutical composition according to claim 1, wherein the human mesenchymal stem cell was passage-cultured twice to 15 times.

[FIG. 1a]

[FIG. 1b]

[FIG. 1c]

| Markers | Percentage |
|---|---|
| S100$^+$ | 44.89 ± 4.12 |
| GFAP$^+$ | 40.41 ± 4.45 |
| S100$^+$/GFAP$^+$ | 40.41 ± 4.45 |

[FIG. 2]

[FIG. 3]

[FIG. 4a]

[FIG. 4b]

[FIG. 5a]

[FIG. 5b]

[FIG. 6a]

[FIG. 6b]

[FIG. 6c]

**p<0.01 by ANOVA and Tukey's post hoc vs Control
***p<0.0001 by ANOVA and Tukey's post hoc vs Control

[FIG. 7a]

[FIG. 7b]

[FIG. 8a]

**Acquisition**

- G0:WT, G1:Control, G2:Low, G3:Medium, G4:High
- G0;n=9, G1; n=10, G2;n=9, G3;n=10, G4;n=9
- *p≤0.05, ***p≤0.001 vs G1
- #p≤0.05 vs G2
- Analysis by One-way ANOVA and post-hoc Tukey's HSD

[FIG. 8b]

**Reversal**

- G0:WT, G1:Control, G2:Low, G3:Medium, G4:High
- G0;n=9, G1; n=10, G2;n=9, G3;n=10, G4;n=9
- *p≤0.05, **p≤0.01, ***p≤0.001 vs G1
- Analysis by One-way ANOVA and post-hoc Tukey's HSD

[FIG. 8c]

## probe trial

- G0:WT, G1:Control, G2:Low, G3:Medium, G4:High
- G0;n=9, G1; n=10, G2;n=9, G3;n=10, G4;n=9
- *p≤0.01 G0 vs G1
- #p≤0.01 G1 vs G4
- Analysis by One-way ANOVA and post-hoc Duncan's MRT

[FIG. 9a]

**Acquisition**

- G0:WT, G1:Control, G2:hMSC, G3:ghMSC
- G0;n=3, G1;n=4, G2;n=4, G3;n=6
- *p<0.05 by One-way ANOVA and post-hoc Duncan's MRT

[FIG. 9b]

Reversal

- G0:WT, G1:Control, G2:hMSC, G3:ghMSC
- G0;n=3, G1;n=4, G2;n=4, G3;n=6
- *p<0.05 by One-way ANOVA and post-hoc Duncan's MRT

[FIG. 9c]

Probe trial

- G0:WT, G1:Control, G2:hMSC, G3:ghMSC
- G0;n=3, G1;n=4, G2;n=4, G3;n=6
- *p<0.05 by One-way ANOVA and post-hoc Duncan's MRT

[FIG. 10]

[FIG. 11a]

## Astrocyte

94.9% expression

[FIG. 11b]

# ghMSC

## BD FACSDiva 9.0

66% expression

[FIG. 12a]

[FIG. 12b]

EP 4 353 244 A1

| | Neurobasal only | hMSC | ghMSC |
|---|---|---|---|
| GRO a/b/g | 1 | 2.52 | 9.37 |
| GRO alpha (CXCL1) | 1 | 2.61 | 5.73 |
| IL-6 | 1 | 9.86 | 5.89 |
| IL-8 | 1 | 5.93 | 14.18 |
| MCP1(CCL2) | 1 | 10.45 | 10.19 |
| VEGF-A | 1 | 2.19 | 2.46 |
| BDNF | 1 | 2.40 | 2.40 |
| HGF | 1 | 3.09 | 2.30 |
| IGFBP-4 | 1 | 1.15 | 1.92 |
| OPG(TNFRSF11) | 1 | 8.32 | 2.23 |
| TIMP1 | 1 | 5.20 | 3.05 |
| TIMP2 | 1 | 13.98 | 10.36 |

[FIG. 12c]

[FIG. 13a]

0hr
3D treatment

+6hr
NB & CM
reoxygenation

+12hr
Cell counting
& Protein prep #1

+18hr
Cell counting
& Protein prep #2

[FIG. 13b]

| | | | | | | |
|---|---|---|---|---|---|---|
| OGD (6hr) | - | + | + | - | + | + |
| NB reoxygenation (6hr) | - | + | - | - | - | - |
| CM reoxygenation (6hr) | - | - | + | - | - | - |
| NB reoxygenation (12hr) | - | - | - | - | + | - |

[FIG. 13c]

[FIG. 13d]

Trypan blue exclusion assay

[FIG. 13e]

Claudin-5

| | | |
|---|---|---|
| OGD (6hr) | - | + | + |
| ghMSC-CM | - | - | + |

Day 0　　　　　Day 4　　　　　Day 7　　　　　Day 8　　　　　Day 12

Brain Slice Culture　　OGD 1 h　　　Reoxygenation 72 h　　Observation of cell death　　ghMSC T.P DIV5
P7 Rat thickness : 350 μm　(95% N2, 5% CO2 gas)

ghMSC T.P
(3x10^4cells/1.5ul)

PI Staining
IHC

[FIG. 14a]

[FIG. 14b]

## Day 4 slice culture

## Day 7 slice culture

### Control        OGD/R

[FIG. 14c]

## Hippocampus

Control        OGD        OGD+hMSC T.P        OGD+ ghMSC T.P

[FIG. 14d]

# Hippocampus

[FIG. 15a]

[FIG. 15b]

[FIG. 16]

GLAST: Glial and aspartate transporter  GFAP: Glial fibrillary acidic protein  VEGF: Vascular endothelial growth factor  PAPP-A: Pregnancy associated plasma protein A
HGF: Hepatocyte growth factor  IGFBP4: Insulin growth factor binding protein-4

| INTERNATIONAL SEARCH REPORT | International application No. |
| --- | --- |
| | **PCT/KR2023/006022** |

| A. CLASSIFICATION OF SUBJECT MATTER |
| --- |
| **A61K 35/28**(2006.01)i; **A61K 35/30**(2006.01)i; **A61P 25/28**(2006.01)i; **C12N 5/079**(2010.01)i |
| According to International Patent Classification (IPC) or to both national classification and IPC |

| B. FIELDS SEARCHED |
| --- |
| Minimum documentation searched (classification system followed by classification symbols) |
| A61K 35/28(2006.01); A61K 35/30(2006.01) |
| Documentation searched other than minimum documentation to the extent that such documents are included in the fields searched |
| Korean utility models and applications for utility models: IPC as above<br>Japanese utility models and applications for utility models: IPC as above |
| Electronic data base consulted during the international search (name of data base and, where practicable, search terms used) |
| eKOMPASS (KIPO internal) & keywords: 유사교세포(glia-like cell), 인간 중간엽 줄기세포(human mesenchymal stem cell), 혈관성 치매(vascular dementia), 치료(treatment) |

| C. DOCUMENTS CONSIDERED TO BE RELEVANT |
| --- |

| Category* | Citation of document, with indication, where appropriate, of the relevant passages | Relevant to claim No. |
| --- | --- | --- |
| Y | KR 10-2021-0023781 A (SEOUL NATIONAL UNIVERSITY R&DB FOUNDATION et al.) 04 March 2021 (2021-03-04)<br>See paragraphs [0010], [0013], [0051], [0053], [0061] and [0078]. | 1-4 |
| Y | LLORENTE, Irene L. et al. Patient-derived glial enriched progenitors repair functional deficits due to white matter stroke and vascular dementia in rodents. Science Translational Medicine. 2021, vol. 13, eaaz6747, pp. 1-17.<br>See pages 1, 4, 10 and 12. | 1-4 |
| Y | SON, Jeong-Woo et al. Glia-like cells from late-passage human MSCs protect against ischemic stroke through IGFBP-4. Molecular Neurobiology. 2019, vol. 56, no. 11, pp. 7617-7630 (inner pp. 1-14).<br>See abstract; and pages 2, 8, 10 and 13. | 1-4 |
| A | WO 2021-034164 A1 (SEOUL NATIONAL UNIVERSITY R&DB FOUNDATION et al.) 25 February 2021 (2021-02-25)<br>See entire document. | 1-4 |

| ☑ Further documents are listed in the continuation of Box C. | ☑ See patent family annex. |
| --- | --- |

| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
| --- | --- |

| Date of the actual completion of the international search | Date of mailing of the international search report |
| --- | --- |
| **07 August 2023** | **07 August 2023** |
| Name and mailing address of the ISA/KR<br><br>**Korean Intellectual Property Office**<br>**Government Complex-Daejeon Building 4, 189 Cheongsa-ro, Seo-gu, Daejeon 35208** | Authorized officer |
| Facsimile No. **+82-42-481-8578** | Telephone No. |

Form PCT/ISA/210 (second sheet) (July 2022)

## INTERNATIONAL SEARCH REPORT

International application No.

**PCT/KR2023/006022**

**C.      DOCUMENTS CONSIDERED TO BE RELEVANT**

| Category* | Citation of document, with indication, where appropriate, of the relevant passages | Relevant to claim No. |
|---|---|---|
| A | WANG, Fei et al. Synergistic effects of mesenchymal stem cell transplantation and repetitive transcranial magnetic stimulation on promoting autophagy and synaptic plasticity in vascular dementia. Journals of Gerontology: BIOLOGICAL SCIENCES. 2019, vol. 74, no. 9, pp. 1341-1350.<br>        See entire document. | 1-4 |

Form PCT/ISA/210 (second sheet) (July 2022)

| **INTERNATIONAL SEARCH REPORT** | International application No. |
|---|---|
| | **PCT/KR2023/006022** |

| **Box No. I** Nucleotide and/or amino acid sequence(s) (Continuation of item 1.c of the first sheet) |
|---|

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

   ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

### INTERNATIONAL SEARCH REPORT
Information on patent family members

International application No.

**PCT/KR2023/006022**

| Patent document cited in search report | | | Publication date (day/month/year) | Patent family member(s) | | | Publication date (day/month/year) |
|---|---|---|---|---|---|---|---|
| KR | 10-2021-0023781 | A | 04 March 2021 | KR | 10-2503876 | B1 | 27 February 2023 |
| | | | | US | 2022-0331368 | A1 | 20 October 2022 |
| | | | | WO | 2021-034162 | A1 | 25 February 2021 |
| WO | 2021-034164 | A1 | 25 February 2021 | KR | 10-2021-0023782 | A | 04 March 2021 |
| | | | | KR | 10-2408550 | B1 | 14 June 2022 |
| | | | | US | 2022-0339198 | A1 | 27 October 2022 |

Form PCT/ISA/210 (patent family annex) (July 2022)